# EUROPÄISCHE PATENTSCHRIFT

(11) **EP 1 902 030 B1**
(45) Veröffentlichungstag und Bekanntmachung des Hinweises auf die Patenterteilung: **11.04.2012**
(21) Anmeldenummer: 06754586.3
(22) Anmeldetag: 27.06.2006
(51) Int. Cl.: C07D 215/56, A61K 31/47, A61P 31/12

(54) **SUBSTITUIERTE CHINOLONE II**
SUBSTITUTED QUINOLONES II
QUINOLONES SUBSTITUEES (II)

(30) Priorität: 30.06.2005 DE 102005030524
(43) Veröffentlichungstag der Anmeldung: 26.03.2008
(73) Patentinhaber: AiCuris GmbH & Co. KG, 42117 Wuppertal (DE)
(72) Erfinder: SCHOHE-LOOP, Rudolf, 42327 Wuppertal (DE); ZIMMERMANN, Holger, 42111 Wuppertal (DE); HENNINGER, Kerstin, 42115 Wuppertal (DE); LANG, Dieter, 42553 Velbert (DE); THEDE, Kai, 42115 Wuppertal (DE); FÜRSTNER, Chantal, 45478 Mülheim an der Ruhr (DE); BRÜCKNER, David, 45128 Essen (DE)
(74) Vertreter: Weller, Wolfgang
(86) Internationale Anmeldenummer: PCT/EP2006/006197
(87) Internationale Veröffentlichungsnummer: WO 2007/003308

(56) Entgegenhaltungen:
- EP-A- 0 276 700
- EP-A1- 0 612 731
- WO-A-00/40561
- WO-A-2006/008046
- WO-A2-02/085886

## Beschreibung

Die Erfindung betrifft substituierte Chinolone und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

WO 00/040561 und US 4,959,363 beschreiben Chinolone mit Wirkung gegen Viren der Herpes-Familie. EP-A 612731 beschreibt Chinolone als antivirale Mittel, besonders gegen HIV. WO 02/009758, WO02/085886 und WO03/050107 beanspruchen Chinolone als Breitspektrum Antibiotika. In WO 97/004775 und WO 97/004779 werden Chinolone als Inhibitoren von PDE4 und TNFα unter anderem für die Behandlung von antiinflammatorischen Erkrankungen und HIV beschrieben. EP-A 276700 beschreibt 8-Cyano-Chinolone als Antibiotika. WO02/026713 beschreibt Chinolone als antiparasitäre Verbindungen.

Auf dem Markt sind strukturell andersartige, antiviral wirkende Mittel vorhanden, deren Anwendungsbreite aufgrund eines ausgeprägten Nebenwirkungsprofils und möglicher Resistenzentwicklung stark eingeschränkt ist. Neue Mittel für eine bessere und wirksamere Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Chinolone antiviral wirksam sind.

Gegenstand der Erfindung sind Verbindungen der Formel 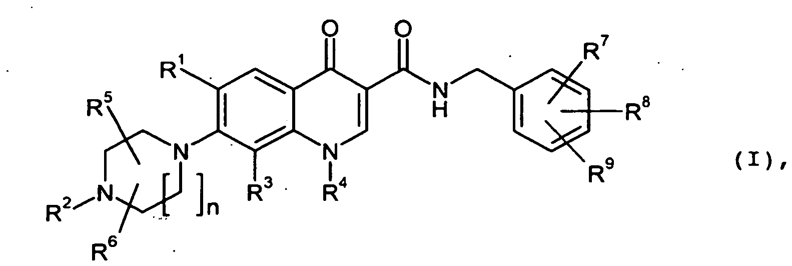 in welcher
- n: für eine Zahl 1 oder 2 steht,
- R¹: für Wasserstoff, Fluor, Chlor oder Trifluormethyl steht,
- R²: für C₁-C₆-Alkyl, C₁-C₆-Alkylaminocarbonyl oder -C(=O)-R¹⁰ steht,
wobei Alkyl und Alkylaminocarbonyl substituiert sind mit einem Substituenten, wobei der Substituent ausgewählt wird aus der Gruppe, bestehend aus Hydroxycarbonyl und C₁-C₆-Alkoxycarbonyl,
und
R¹⁰ für Hydroxycarbonyl, C₁-C₆-Alkoxycarbonyl, Hydroxycarbonylmethyl oder C₁-C₆-Alkoxycarbonylmethyl steht,
- R³: für Halogen, Cyano, Methoxy, Trifluormethyl, Monofluormethoxy, Difluormethoxy, Trifluormethoxy oder Ethinyl steht,
- R⁴: für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkoxy, C₁-C₆-Alkylamino, C₁-C₆-Alkylcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
oder
- R³ und R⁴: bilden gemeinsam mit den Atomen an die sie gebunden sind einen Ring durch eine Gruppe der Formel 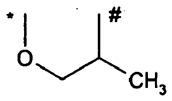 wobei
* die Anknüpfstelle an das Kohlenstoffatom ist,
und
# die Anknüpfstelle an das Stickstoffatom ist,
- R⁵ und R⁶: unabhängig voneinander für Wasserstoff, Methyl oder Ethyl stehen,
- R⁷ und R⁸: unabhängig voneinander für Halogen, Hydroxy, Cyano, Trifluormethyl, Monofluormethoxy, Difluormethoxy, Trifluormethoxy, C₁-C₃-Alkyl oder C₁-C₃-Alkoxy stehen,
- R⁹: für Wasserstoff, Halogen, Hydroxy, Cyano, Trifluormethyl, Monofluormethoxy, Difluormethoxy, Trifluormethoxy, C₁-C₃-Alkyl oder C₁-C₃-Alkoxy steht,
und ihre Salze, ihre Solvate und die Solvate ihrer Salze.

Erfindungsgemäße Verbindungen sind die Verbindungen der Formel (I), (Ia) und (Ib) und deren Salze, Solvate und Solvate der Salze; die von Formel (I), (Ia) und (Ib) umfassten Verbindungen der nachfolgend genannten Formeln und deren Salze, Solvate und Solvate der Salze sowie die von Formel (I), (Ia) und (Ib) umfassten, nachfolgend als Ausführungsbeispiele genannten Verbindungen und deren Salze, Solvate und Solvate der Salze, soweit es sich bei den von Formel (I), (Ia) und (Ib) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium-und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylcarbonyl, Alkoxycarbonyl und Alkylaminocarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert-*Butyl, n-Pentyl und n-Hexyl.

Alkoxy steht beispielhaft und vorzugsweise für Methoxy, Ethoxy, n-Propoxy, Isopropoxy, *tert*-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N,N*-Dimethylamino, *N,N*-Diethylamino, *N*-Ethyl-*N-*methylamino, *N*-Methyl-*N*-n-propylamino, *N*-Isopropyl-*N*-n-propylamino, *N-tert*-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylcarbonyl steht beispielhaft und vorzugsweise für Acetyl und Propanoyl.

Alkoxycarbonyl steht beispielhaft und vorzugsweise für Methoxycarbonyl, Ethoxycarbonyl, n-Propoxycarbonyl, Isopropoxycarbonyl, tert-Butoxycarbonyl, n-Pentoxycarbonyl und n-Hexoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*-Butylaminocarbonyl, n-Pentylaminocarbonyl, n-Hexylaminocarbonyl, *N,N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propyl-aminocarbonyl, *N-tert*-Butyl-*N*-methylaminocarbonyl, *N*-Ethyl-*N*-n-pentylaminocarbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Cycloalkyl steht für eine Cycloalkylgruppe mit in der Regel 3 bis 8, bevorzugt 3 bis 5 Kohlenstoffatomen, beispielhaft und vorzugsweise für Cycloalkyl sind genannt Cyclopropyl, Cyclobutyl, Cyclopentyl, Cyclohexyl und Cycloheptyl.

Halogen steht für Fluor, Chlor, Brom und Jod, bevorzugt Fluor und Chlor.

In der Formel der Gruppe, die für R³ und R⁴ stehen kann, steht der Endpunkt der Linie, neben der jeweils ein * oder # steht, nicht für ein Kohlenstoffatom beziehungsweise eine CH₂-Gruppe sondern ist Bestandteil der Bindung zu dem Atom, an das R³ und R⁴ gebunden sind.

Bevorzugt sind solche Verbindungen der Formel (I), welche der Formel 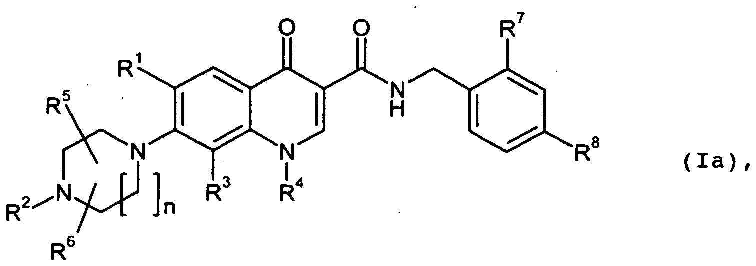 entsprechen,
in welcher
- n: für die Zahl 1 steht,
- R¹: für Wasserstoff oder Fluor steht,
- R²: für C₁-C₄-Alkyl steht,
wobei Alkyl substituiert ist mit einem Substituenten, wobei der Substituent ausgewählt wird aus der Gruppe, bestehend aus Hydroxycarbonyl und C₁-C₆-Alkoxycarbonyl,
- R³: für Fluor, Chlor, Trifluormethyl, Methoxy, Monofluormethoxy, Difluormethoxy, Trifluormethoxy oder Ethinyl steht,
- R⁴: für C₁-C₄-Alkyl oder C₃-C₅-Cycloalkyl steht,
wobei Alkyl substituiert sein kann mit 1 bis 3 Substituenten, wobei die Substituenten unabhängig voneinander ausgewählt werden aus der Gruppe, bestehend aus Fluor, Hydroxy und C₁-C₃-Alkoxy, und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Trifluormethyl, C₁-C₃-Alkyl und C₁-C₃-Alkoxy,
oder
- R³ und R⁴: bilden gemeinsam mit den Atomen an die sie gebunden sind einen Ring durch eine Gruppe der Formel 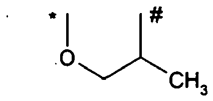 wobei
* die Anknüpfstelle an das Kohlenstoffatom ist, und
# die Anknüpfstelle an das Stickstoffatom ist,
- R⁵ und R⁶: unabhängig voneinander für Wasserstoff oder Methyl stehen,
- R⁷ und R⁸: unabhängig voneinander für Fluor, Chlor, Cyano, Trifluormethyl, Difluormethoxy, Trifluormethoxy, C₁-C₃-Alkyl oder C₁-C₃-Alkoxy stehen,
und ihre Salze, ihre Solvate und die Solvate ihrer Salze.

Bevorzugt sind besonders solche Verbindungen der Formel (I) oder (Ia), welche der Formel 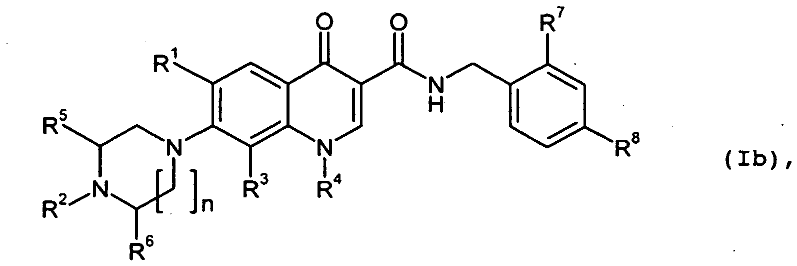 entsprechen,
in welcher
- n: für die Zahl 1 steht,
- R¹: für Fluor steht,
- R²: für Methyl oder Ethyl steht,
wobei Methyl und Ethyl substituiert sind mit einem Substituenten, wobei der Substituent ausgewählt wird aus der Gruppe, bestehend aus Hydroxycarbonyl, Methoxycarbonyl und Ethoxycarbonyl,
- R³: für Chlor, Methoxy, Difluormethoxy oder Trifluormethoxy steht,
- R⁴: für Methyl, Ethyl oder Cyclopropyl steht, wobei Ethyl substituiert sein kann mit 1 bis 3 Substituenten Fluor,
und
wobei Cyclopropyl substituiert sein kann mit 1 bis 2 Substituenten Fluor,
oder
- R³ und R⁴: bilden gemeinsam mit den Atomen an die sie gebunden sind einen Ring durch eine Gruppe der Formel 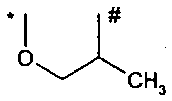 wobei
* die Anknüpfstelle an das Kohlenstoffatom ist, und
# die Anknüpfstelle an das Stickstoffatom ist,
- R⁵ und R⁶: unabhängig voneinander für Wasserstoff oder Methyl stehen,
- R⁷ und R⁸: unabhängig voneinander für Chlor, Trifluormethyl, Trifluormethoxy oder Methyl stehen,
und ihre Salze, ihre Solvate und die Solvate ihrer Salze.

Bevorzugt sind auch solche Verbindungen der Formel (I), (Ia) und (Ib), in welcher R¹ für Fluor steht.

Bevorzugt sind auch solche Verbindungen der Formel (I), (Ia) und (Ib), in welcher R² für Methylen steht, wobei Methylen substituiert ist mit einem Substituenten, wobei der Substituent ausgewählt wird aus der Gruppe, bestehend aus Hydroxycarbonyl, Methoxycarbonyl und Ethoxycarbonyl.

Bevorzugt sind auch solche Verbindungen der Formel (I), (Ia) und (Ib), in welcher R³ für Chlor, Methoxy oder Difluormethoxy steht.

Bevorzugt sind auch solche Verbindungen der Formel (I), (Ia) und (Ib), in welcher R⁴ für Cyclopropyl oder 2-Fluor-cycloprop-1-yl steht.

Bevorzugt sind auch solche Verbindungen der Formel (I), (Ia) und (Ib), in welcher R⁴ für 2,2,2-Trifluorethyl steht.

Bevorzugt sind auch solche Verbindungen der Formel (I), (Ia) und (Ib), in welcher R⁷ und R⁸ für Chlor stehen.

Bevorzugt sind auch solche Verbindungen der Formel (I), (Ia) und (Ib), in welcher R⁷ für Chlor oder Methyl und R⁸ für Trifluormethyl oder Trifluormethoxy steht.

Bevorzugt sind auch solche Verbindungen der Formel (I), in welcher R⁹ für Wasserstoff steht.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei Verbindungen der Formel 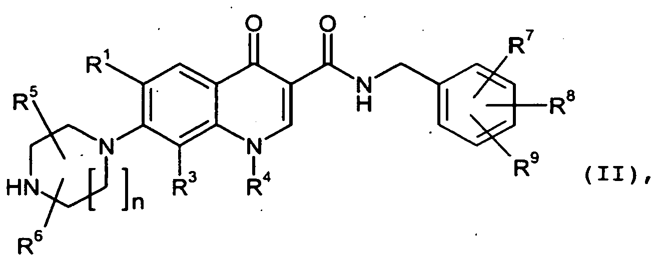 in welcher
n, R¹, R³ R⁴, R⁵, R⁶, R⁷, R⁸ und R⁹ die oben angegebene Bedeutung aufweisen,
nach Verfahren [A] mit Verbindungen der Formel

R²-X¹ (III),

in welcher
- R²: für C₁-C₆-Alkyl steht,
wobei Alkyl substituiert ist mit einem Substituenten C₁-C₆-Alkoxycarbonyl,
und
- X¹: für Halogen, bevorzugt Iod, Chlor oder Brom, oder Mesylat, Tosylat oder Triflat steht,
oder
nach Verfahren [B] mit Verbindungen der Formel

R^{2a}-NCO (IV),

in welcher
- R^{2a}: für das Alkyl von Alkylaminocarbonyl des Restes R² steht,
wobei Alkylaminocarbonyl substituiert ist mit einem Substituenten C₁-C₆-Alkoxycarbonyl,
oder
nach Verfahren [C] mit Verbindungen der Formel 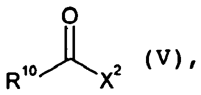 in welcher
- R¹⁰: die oben angegebene Bedeutung aufweist, und
- X²: für Halogen, bevorzugt Chlor oder Brom steht,
umgesetzt werden,
oder
wobei Verbindungen, die durch die Umsetzung von Verbindungen der Formel (II) mit Verbindungen der Formeln (III) oder (IV) entstehen,
nach Verfahren [D] mit einer Base zu der entsprechenden Säure verseift werden.

Die Umsetzung nach Verfahren [A] erfolgt im Allgemeinen in inerten Lösungsmitteln, in Gegenwart einer Base, bevorzugt in einem Temperaturbereich von -30°C bis 120°C bei Normaldruck.

Inerte Lösungsmittel sind beispielsweise Halogenkohlenwasserstoffe wie Dichlormethan oder Trichlormethan, Kohlenwasserstoff wie Benzol, oder andere Lösungsmittel wie Nitromethan, Dioxan, Dimethylformamid oder Acetonitril. Ebenso ist es möglich, Gemische der Lösemittel einzusetzen. Besonders bevorzugt ist Acetonitril, Dichlormethan oder Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin oder Diisopropylethylamin.

Die Umsetzung nach Verfahren [B] erfolgt im Allgemeinen in inerten Lösungsmitteln, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, oder andere Lösungsmittel wie Nitromethan, Dioxan, Dimethylformamid oder Acetonitril. Ebenso ist es möglich,. Gemische der Lösemittel einzusetzen. Besonders bevorzugt ist Dichlormethan oder Dimethylformamid.

Die Umsetzung nach Verfahren [C] erfolgt im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, oder andere Lösungsmittel wie Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise Alkalicarbonate, wie z.B. Natrium- oder Kaliumcarbonat, oder -hydrogencarbonat, oder organische Basen wie Pyridin oder Trialkylamine z.B. Triethylamin, *N*-Methylmorpholin, *N*-Methylpiperidin, 4-Dimethylaminopyridin oder Diisopropylethylamin.

Die Verseifung nach Verfahren [D] erfolgt im Allgemeinen in Wasser oder inerten Lösungsmitteln oder Mischungen von Wasser und inerten Lösungsmitteln, in Gegenwart einer Base, bevorzugt in einem Temperaturbereich von -30°C bis 100°C bei Normaldruck.

Inerte Lösungsmittel sind beispielsweise Halogenkohlenwasserstoffe wie Dichlormethan oder Trichlormethan, Kohlenwasserstoff wie Benzol, oder andere Lösungsmittel wie Nitromethan, Dioxan, Methanol, Tetrahydrofuran, Dimethylformamid oder Acetonitril. Ebenso ist es möglich, Gemische der Lösemittel einzusetzen. Besonders bevorzugt ist Dioxan, Methanol, Tetrahydrofuran oder Dimethylformamid.

Basen sind beispielsweise Alkalihydroxide oder -carbonate, wie z.B. Natrium-, Kalium- oder Lithiumhydroxid, Natrium- oder Kaliumcarbonat, oder -hydrogencarbonat.

Die Verbindungen der Formeln (III), (IV) und (V) sind bekannt oder lassen sich nach bekannten Verfahren aus den entsprechenden Edukten synthetisieren.

Die Verbindungen der Formel (I), in welcher R² für ein substituiertes Ethyl steht, können auch durch die Umsetzung der Verbindungen der Formel (II) mit einem Michael-Akzeptor, wie z. B. Acrylsäureethylester, hergestellt werden.

Die Verbindungen der Formel (II) sind bekannt oder können hergestellt werden, indem Verbindungen der Formel 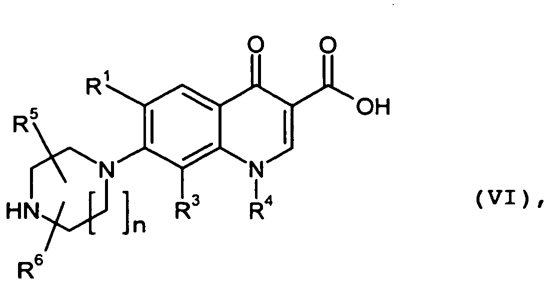 in welcher
n, R¹, R³, R⁴, R⁵ und R⁶ die oben angegebene Bedeutung aufweisen,
mit Verbindungen der Formel 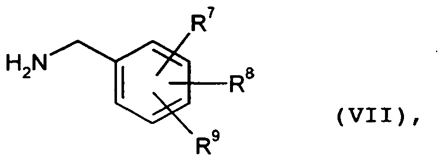 in welcher
R⁷, R⁸ und R⁹ die oben angegebene Bedeutung aufweisen,
umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, N-Methylpiperidin, 4-Dimethylaminopyridin oder Diisopropylethylamin.

Als Dehydratisierungsreagenzien eignen sich hierbei beispielsweise Carbodiimide wie z.B. *N,N*'-Diethyl-, *N,N,'*-Dipropyl-, *N,N'*-Diisopropyl-, *N,N'*-Dicyclohexylcarbodiimid, *N*-(3-Di-methylaminoisopropyl)-*N*'-ethylcarbodiimid-hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid, oder O-(Benzotriazol-1-yl)-*N,N,N',N'*-tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Benzotriazol-1-yloxytris(pyrrolidino)-phosphoniumhexafluorophosphat (PyBOP), oder *N-*Hydroxysuccinimid, oder Mischungen aus diesen, mit Basen. Vorzugsweise wird die Kondensation mit HATU, Benzotriazol-1-yloxytris(pyrrolidino)-phosphoniumhexafluorophosphat (PyBOP) oder mit EDC in Gegenwart von HOBt durchgeführt.

Die Verbindungen der Formel (VII) sind bekannt oder lassen sich nach bekannten Verfahren aus den entsprechenden Edukten synthetisieren.

Die Verbindungen der Formel (VI) sind bekannt oder können hergestellt werden, indem Verbindungen der Formel 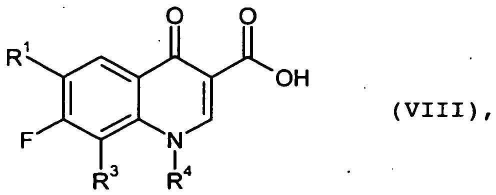 in welcher
R¹, R³ und R⁴ die oben angegebene Bedeutung aufweisen,
mit Verbindungen der Formel 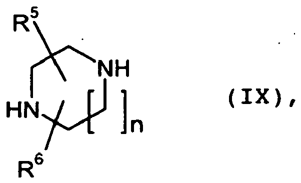 in welcher
n, R⁵ und R⁶ die oben angegebene Bedeutung aufweisen,
umgesetzt werden.

Die Umsetzung kann nach den in A. Da Silva, M. De Almeida, V. De Souza, M. Couri, Current Medicinal Chemistry, 2003, 10, 21-39 beschriebenen Verfahren durchgeführt werden.

Bei der Umsetzung der Verbindungen der Formel (VIII) mit Verbindungen der Formel (IX) ist gegebenenfalls eines der Stickstoffatome mit einer Schutzgruppe, wie z. B. Fmoc oder Boc geschützt, die nach der Umsetzung nach dem Fachmann bekannten Bedingungen abgespalten wird.

Die Verbindungen der Formel (VIII) sind bekannt oder lassen sich nach bekannten Verfahren aus den entsprechenden Edukten synthetisieren, wie z.B. in A. Da Silva, M. De Almeida, V. De Souza, M. Couri, Current Medicinal Chemistry, 2003, 10, 21-39 beschrieben.

Die Verbindungen der Formel (IX) sind bekannt oder lassen stich nach bekannten Verfahren aus den entsprechenden Edukten synthetisieren.

In einem alternativen Verfahren können zur Herstellung der Verbindungen der Formel (II) die nukleophilische Substitution an der 7-Position des Chinolons und die Amidbildung in der Reihenfolge der Umsetzung vertauscht werden.

Die Herstellung der erfindungsgemäßen Verbindungen kann durch das folgende Syntheseschema verdeutlicht werden.

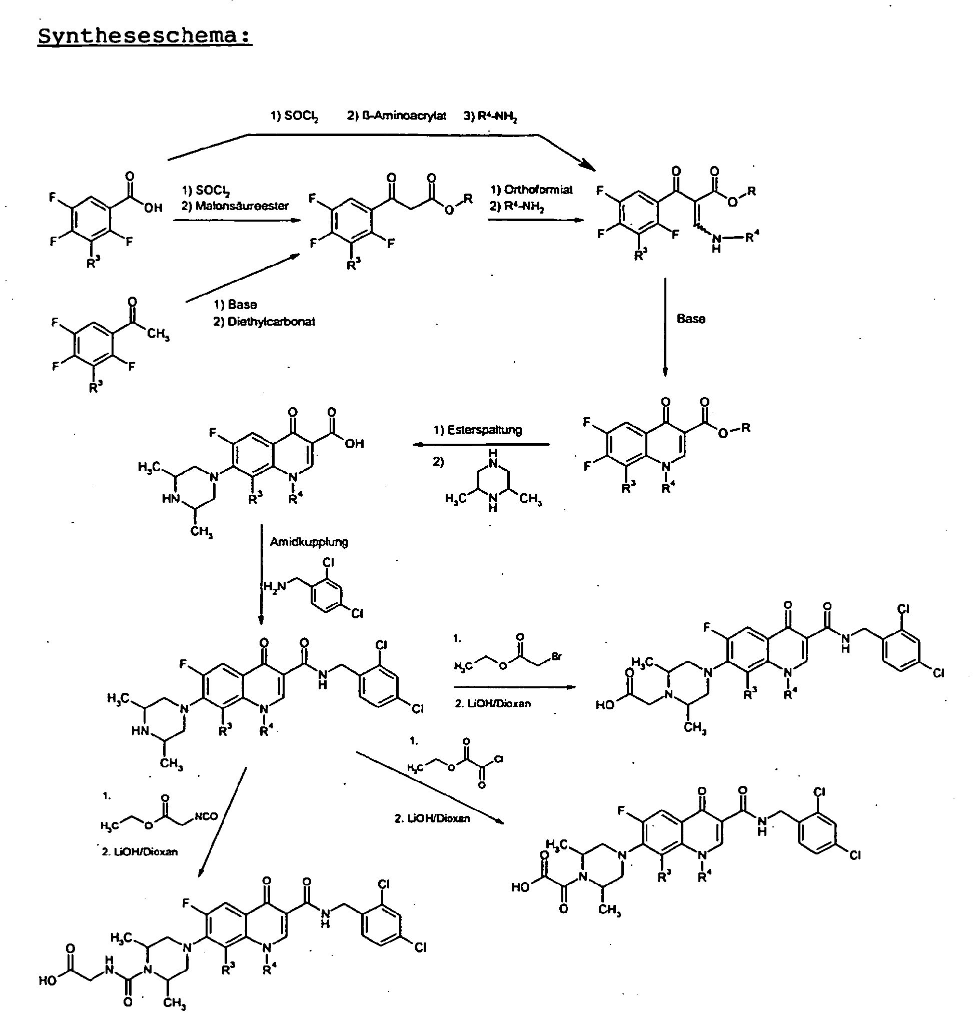

Die erfindungsgemäßen Verbindungen zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.
6) Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung ist der Einsatz zur Behandlung und/oder Prophylaxe von Erkrankungen, insbesondere der zuvor genannten Erkrankungen, unter Verwendung einer antiviral wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Valgancyclovir, Gancyclovir oder Acyclovir.

Die erfindungsgemäßen Verbindungen können systemisch und/oder lokal wirten. Zu diesem Zweck können sie auf geeignete Weise appliziert werden, wie z.B. oral, parenteral, pulmonal, nasal, sublingual, lingual, buccal, rectal, dermal, transdermal, conjunctival, otisch, topisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder a-morphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- ca.: circa
- Boc: tert-Butoxycarbonyl
- CDCl₃: Deuterochloroform
- DCI: direkte chemische Ionisation (bei MS)
- DCM: Dichlormethan
- DIEA: *N,N*-Diisopropylethylamin
- DMSO: Dimethylsulfoxid
- DMF: *N,N*-Dimethylformamid
- d. Th.: der Theorie
- EDC: *N*-(3-Dimethylaminoisopropyl)-*N*'-ethylcarbodiimid-hydrochlorid
- EE: Ethylacetat (Essigsäureethylester)
- EI: Elektronenstoß-Ionisation (bei MS)
- ESI: Elektrospray-Ionisation (bei MS)
- Fmoc: 9-Fluorenylmethoxycarbonyl
- ges.: gesättigt
- ggfs.: gegebenenfalls
- H: Stunde
- HPLC: Hochdruck-, Hochleistungsflüssigchromatographie
- HV: Hochvakuum
- konz.: konzentriert
- LC-MS: Flüssigchromatographie-gekoppelte Massenspektroskopie
- LDA: Lithium-Diisopropylamid
- min.: Minuten
- MS: Massenspektroskopie
- MTBE: Methyl-tert-butylether
- NMR: Kernresonanzspektroskopie
- Pd-C: Palladium auf Kohle
- proz.: prozentig
- PyBOP: 1-Benzotriazolyloxy-tripyrrolidinophosphoniumhexafluorophosphat
- RP-HPLC: Reverse Phase HPLC
- RT: Raumtemperatur
- Rₜ: Retentionszeit (bei HPLC)
- THF: Tetrahydrofuran

### Allgemeine Methoden LC-MS und HPLC :

**Methode 1 (LC-MS) :** Instrument: Micromass Quattro LCZ mit HPLC Agilent Serie 1100 ; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 2 (LC-MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 3 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (präparative HPLC):** Säule: RP18; Gradient unter Zusatz von 0.2% diethylamin zum Acetonitril: 30% Acetonitril / 70% Wasser → 95% Acetonitril / 5% Wasser.

**Methode 5 (präparative HPLC, Ameisensäure):** Säule: Grom-Sil 120 ODS-4HE, 10 µm, SNr. 3331, 250 mm x 30 mm. Eluent A: Ameisensäure 0.1% in Wasser, Eluent B: Acetonitril; Fluss: 50 ml/min. Programm: 0-3 min : 10% B; 3-27 min : Gradient bis 95% B; 27-34 min : 95% B; 34.01-38 min: 10% B.

**Methode 6 (präparative HPLC, Salzsäure):** Säule: Grom-Sil 120 ODS-4HE, 10 µm, SNr. 3331, 250 mm x 30 mm. Eluent A: Salzsäure 0.1% in Wasser, Eluent B: Acetonitril; Fluss: 50 ml/min. Programm: 0-2 min 10% B, 3-43 min: Gradient bis 100% B, 43.01-45 min: 100% B.

**Methode 7 (präparative HPLC) :** Säule: Grom-Sil 120 ODS-4HE, 10 µm, SNr. 3331, 250 mm x 30 mm. Eluent A: Wasser, Eluent B: Acetonitril, Fluss: 50 ml/min. Programm: 0-3 min: 10% B; 3-27 min: Gradient bis 95% B; 27-34 min: 95% B; 34.01-38 min: 10% B.

**Methode 8 (präparative HPLC, Trifluoressigsäure):** Säule: Grom-Sil 120 ODS-4HE, 10 µm, SNr. 3331, 250 mm x 30 mm. Eluent A: Trifluoressigsäure 0.1% in Wasser, Eluent B: Acetonitril. Fluss: 50 ml/min. Programm: 0-3 min: 10% B; 3-27 min: Gradient bis 95% B; 27-34 min: 95% B; 34.01-38 min: 10% B.

**Methode 9 (analytische HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig) /l Wasser, Eluent B: Acetonitril; Gradient: 0 min 2%B, 0.5 min 2%B, 4.5 min 90%B, 9 min 90%B, 9.2 min 2%B, 10 min 2%B; Fluss: 0.75 ml/min; Säulen-Temperatur: 30°C; UV-Detektion: 210 nm.

**Methode 10 (analytische HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig) /l Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluss: 0.75 ml/min; Säulen-Temperatur: 30°C; UV-Detektion: 210 nm.

**Methode 11 (LC-MS):** Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 0.5 ml 50%ige Ameisensäure / 1; Eluent B: Acetonitril + 0.5 ml 50%ige Ameisensäure / l; Gradient: 0.0 min 10%B→ 7.0 min 95%B → 9.0 min 95%B; Fluss: 0.0 min 1.0 ml/min → 7.0 min 2.0 ml/min → 9.0 min 2.0 ml/min; Ofen: 35°C; W-Detektion : 210 nm.

Die Beispiel-Verbindungen, die einen basischen Stickstoff enthalten, können abhängig von der Reinigungsmethode als freie Base oder in verschiedenen Salzformen isoliert werden. Die Herstellungsmethode beschreibt oft die Reinigung durch HPLC unter Ameisensäure-Zusatz (Methode 5), was zum Hydroformiat führt oder unter Zusatz von anderen Säuren, wie z.B. Salzsäure (Methode 6) statt Ameisensäure, wobei das Produkt als Hydrochlorid isoliert wird. Alternativ kann das Produkt auch durch Verrühren in Acetonitril oder durch präparative HPLC ohne Säure-Zusatz (Methode 7) gereinigt werden, wobei das Produkt als freie Base isoliert wird. Sowohl aus der freien Base wie auch aus dem Hydroformiat kann durch anschließende Versetzung mit Chlorwasserstoff in Dioxan und Abdampfen am Rotationsverdampfer wiederum das Hydrochlorid der Verbindung erhalten werden.

### Ausgangsverbindungen

### Beispiel 1A

### 2-Brom-4-chlor-benzonitril

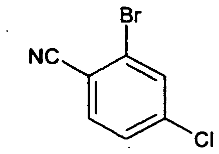

588 mg 2-Brom-4-chlorbenzoesäure und 300 mg Harnstoff werden in Dichlormethan / Methanol gelöst und auf 364 mg Aluminiumoxid einrotiert. Der Rückstand wird in der Mikrowelle bei 150°C insgesamt 60 min bestrahlt. Nach Abkühlung wird mit Ethylacetat und Wasser verrührt, abfiltriert und die wässrige Phase abgetrennt. Die organische Phase wird mit Natriumhydrogencarbonat-Lösung gewaschen, über Natriumsulfat getrocknet, einrotiert und dann im Hochvakuum getrocknet. Das Produkt wird ohne zusätzliche Reinigung weiter umgesetzt.

¹H-NMR (300 MHz, CDCl₃) : δ = 7.72 (d, 1H), 7.60 (d, 1H), 7.42 (dd, 1H).

### Beispiel 2A

### 2-Chlor-4-(trifluormethoxy)-phenyl-trifluormethylsulfonat

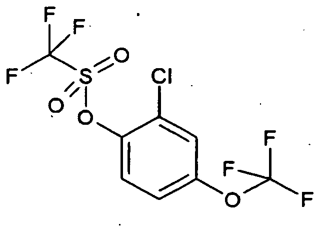

4.00 g 2-Chlor-4-trifluormethoxy-phenol in 50 ml Toluol und 50 ml einer 30%igen Kaliumphosphat-Lösung in Wasser werden bei 0°C vorgelegt, langsam mit 3.82 ml Trifluormethansulfonsäureanhydrid versetzt und 1.5 h bei RT gerührt. Die wässrige Phase wird abgetrennt und die organische Phase mit Wasser gewaschen, über Natriumsulfat getrocknet und eingeengt. Das Rohprodukt wird ohne Reinigung zu Beispiel 3A weiter umgesetzt.

### Beispiel 3A

### 2-Chlor-4-trifluormethoxy-benzonitril

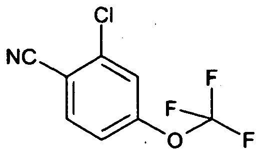

3.00 g der Verbindung aus Beispiel 2A werden in 12 ml entgastem DMF mit 2.04 g Zinkcyanid und 1.00 g Tetrakis(triphenylphosphin)palladium gelöst und unter Argon bei 120°C 2 h erhitzt. Nach Abkühlung wird die Reaktionsmischung mit Ethylacetat verdünnt und zweimal mit gesättigter Natriumhydrogencarbonat-Lösung, dann mit gesättigter Natriumchlorid-Lösung ausgeschüttelt. Die organische Phase wird über Natriumsulfat getrocknet und eingeengt. Der Rückstand wird durch Kieselgel-Chromatographie (Cyclohexan / Ethylacetat 10:1) gereinigt.

¹H-NMR (300 MHz, DMSO-d₆): δ = 7.62 (dd, 1H), 7.95 (d, 1H), 8.18 (d, 1H).

### Beispiel 4A

### 2-Methyl-4-(trifluormethoxy)benzamid

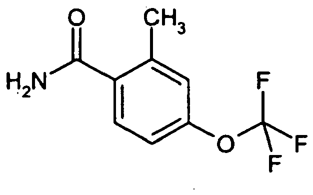

795 mg (3.61 mmol) 2-Methyl-4-(trifluormethoxy)benzoesäure werden mit 4 ml (54.8 mmol) Thionylchlorid und einem Tropfen DMF 30 min unter Rückfluss erhitzt. Nach dem Abkühlen wird die Reaktionslösung langsam in eisgekühlte konz. wässrige Ammoniaklösung getropft. Der entstandene Niederschlag wird abgesaugt, in 30 ml Wasser aufgenommen und 1 h bei 60°C gerührt. Man lässt abkühlen, filtriert den Feststoff ab und trocknet ihn im Vakuum. Ausbeute: 562 mg (71% d. Th.)

LC-MS (Methode 2): Rₜ = 1.61 min.

MS (ESI⁺): m/z = 220 (M+H)⁺

¹H-NMR (400MHz, DMSO-d₆) : δ = 7.79 (bs, 1H), 7.42-7.50 (m, 2H), 7.19-7.28 (m, 2H), 2.39 (s, 3H).

### Beispiel 5A

### 2-Methyl-4-(trifluormethoxy)-benzylamin

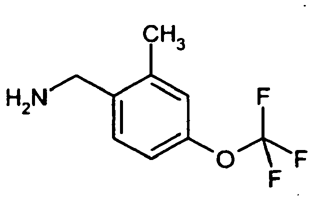

18.8 ml (18.8 mmol) Boran-THF-Komplex (1M) wird unter Argon und Eiskühlung vorgelegt. Es wird eine Lösung von 823 mg (3.76 mmol) 2-Methyl-4-(trifluormethoxy)benzamid (Beispiel 4A) in 80 ml THF zugetropft und anschließend 8 h unter Rückfluss gerührt. Unter Eiskühlung werden 80 ml 1N Salzsäure (bis zur Beendigung der Gasentwicklung) zugetropft und 1 h unter Rückfluss erhitzt. Anschließend wird das Reaktionsgemisch mit IN Natronlauge alkalisch gestellt, dreimal mit Dichlormethan extrahiert, die vereinigten organischen Phasen über Natriumsulfat getrocknet und das Lösungsmittel im Vakuum entfernt. Man erhält ein Öl, das ohne weitere Reinigung weiter umgesetzt wird. Ausbeute: 732 mg (95% d. Th.).

LC-MS (Methode 3): Rₜ = 1.41 min.

MS (ESI⁺) : m/z = 206 (M+H)⁺

¹H-NMR (400MHz, CDCl₃) : δ = 7.32-7.40 (m, 1H), 6.99-7.11 (m, 2H), 3.95-4.01 (m, 2H), 2.40 (s, 3H).

### Beispiel 6A

### 2-Brom-4-chlor-benzylamin

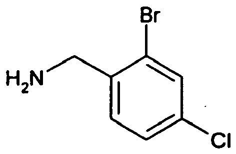

13.9 ml Boran-THF Komplex werden unter Eiskühlung vorgelegt. Langsam wird eine Lösung von 2.0 g 2-Brom-4-chlorbenzonitril (Beispiel 1A) in 60 ml THF dazugegeben. Danach wird die Reaktionsmischung 1 h unter Rückfluss erhitzt, abgekühlt und unter Eiskühlung mit 20 ml 1N Salzsäure tropfenweise versetzt. Man erhitzt 1 h unter Rückfluss und lässt abkühlen. Zur Aufarbeitung wird die Lösung mit 1N Natronlauge alkalisch gestellt und mit Dichlomethan extrahiert. Die organische Phase wird über Natriumsulfat getrocknet und einrotiert. Das Rohprodukt wird ohne Reinigung weiter umgesetzt.

¹H-NMR (300 MHz, CDCl₃): δ = 3.89(s, 2H), 7.35-7.45 (m [ABM], 2H), 7.55 (d, 1H).

### Beispiel 7A

### 2-Chlor-4-trifluormethoxy-benzylamin - Hydrochlorid

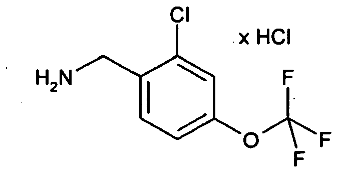

Die Herstellung erfolgt analog zu Beispiel 6A aus der Verbindung aus Beispiel 3A mit anschließender Behandlung mit 4N Salzsäure in Dioxan.

¹H-NMR (300 MHz, DMSO-d₆): δ = 4.15 (s, 2H), 7.52 (d, 1H), 7.70 (s, 1H), 7.78 (d, 1H), 8.56 (bs, 3H).

### Beispiel 8A

### 2,4-Dichlor-6-methyl-benzylamin - Hydrochlorid

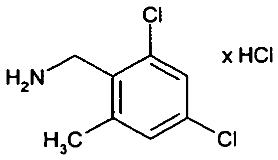

Die Herstellung erfolgt analog zu Beispiel 6A aus 2,4-Dichlor-6-methyl-benzonitril mit anschließender Behandlung mit 4N Salzsäure in Dioxan.

¹H-NMR (300 MHz, DMSO-d₆) : δ = 2.5 (s, 3H), 4.10 (s, 2H), 7.40 (s, 1H), 7.60 (s, 1H), 8.40 (bs, 3H).

LC-MS (Methode 13): Rₜ = 2.44 min, MS (ES+) = 190 (M+H)⁺.

### Beispiel 9A

### 4-Chlor-2-trifluormethyl-benzylamin - Hydrochlorid

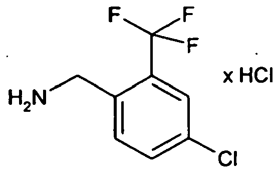

Die Herstellung erfolgt analog zu Beispiel 6A aus 4-Chlor-2-trifluormethyl-benzonitril mit anschließender Behandlung mit 4N Salzsäure in Dioxan.

¹H-NMR (300 MHz, DMSO-d₆): δ = 4.18 (d, 2H), 7.82 (d, 1H), 7.88-7.98 (m, 2H), 8.58 (bs, 3H).

### Beispiel 10A

### 3-[(2,2,2-Trifluorethyl)amino]-2-(2,4,5-trifluor-3-methoxybenzoyl)acrylsäureethylester (E + Z)

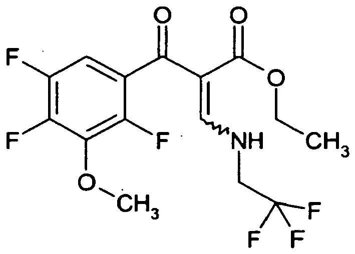

2.00 g (5.79 mmol) 3-Oxo-3-(2,4,5-trifluor-3-methoxyphenyl)-propansäureethylester (Herstellung siehe Journal of Medicinal Chemistry (1995), 38 (22), 4478-87) werden in 3.8 ml (4.14 g, 40.55 mmol) Essigsäureanhydrid und 4.82 ml (4.29 g, 28.96 mmol) Orthoameisen-säuretriethylester für 2 h unter Rückfluss gerührt. Das Lösungsmittel wird anschließend am Rotationsverdampfer vollständig entfernt und der Rückstand in 10 ml Ethanol gelöst. Zur eisgekühlten Lösung werden 1.03 g (10.43 mmol) 2,2,2-Trifluor-1-aminoethan zugetropft, auf Raumtemperatur gebracht und bei dieser Temperatur wird über Nacht nachgerührt. Zur Aufarbeitung wird das Lösungsmittel entfernt und der Rückstand ohne Reinigungsschritte als Rohprodukt weiter umgesetzt.

LC-MS (Methode 2): Rₜ = 2.37 min, MS (ES+) = 386 (M+H)⁺.

Die folgenden Beispiele 11A bis 14A werden analog zu Beispiel 10A aus den entsprechenden Aminen hergestellt.

| **Beispiel-Nr.** | **Struktur** | **Analytikdaten LC-MS (Methode)/ Meßwerte** |
|---|---|---|
| **11A** (S-Enantiomer) | 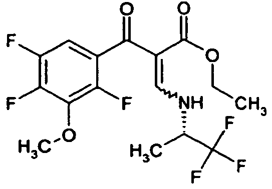 | LC-MS (Methode 1): Rₜ = 2.47 min MS (ES+): m/z = 400 (M+H)⁺ |
| **12A** | 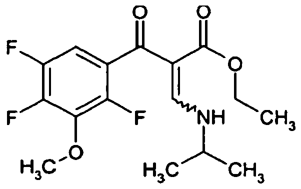 | LC-MS (Methode 1): Rₜ = 2.56 min MS (ES+): m/z = 346 (M+H)⁺ |
| **13A** | 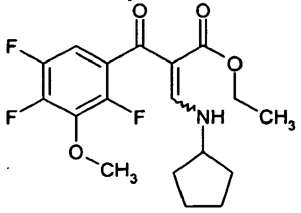 | LC-MS (Methode 1): Rₜ = 2.77 min MS (ES+): m/z = 372 (M+H)⁺ |
| **14A** (1S,2R)-Enantiomer | 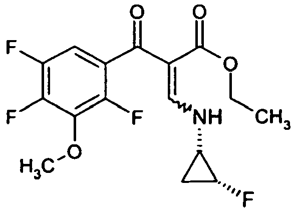 | LC-MS (Methode 1): Rₜ = 2.40 min MS (ES+): m/z = 382 (M+H)⁺ |

### Beispiel 15A

### 6,7-Difluor-8-methoxy-4-oxo-1-(2,2,2-trifluorethyl)-1,4-dihydrochinolin-3-carbonsäureethylester

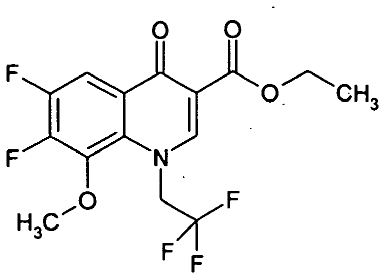

Unter Argon-Atmosphäre und Eiskühlung werden 0.32 g (8.11 mmol) 60%iges Natriumhydrid in 5 ml Tetrahydrofuran vorgelegt und eine Lösung von 2.23 g (5.79 mmol) der Verbindung aus Beispiel 10A in 15 ml Tetrahydrofuran wird langsam zugetropft. Die Mischung wird anschließend auf Raumtemperatur erwärmt, bei dieser Temperatur 2 h nachgerührt und über Nacht stehen gelassen. Zur Aufarbeitung werden 2 ml Essigsäure zugetropft, 5 min nachgerührt, mit Essigsäure-ethylester verdünnt, mehrmals mit Wasser und einmal mit gesättigter Natriumhydrogencarbonat- Lösung gewaschen, die organische Phase über Magnesiumsulfat getrocknet, filtriert und das Lösungsmittel wird vollständig am Rotationsverdampfer entfernt. Das Rohprodukt wird säulenchromatographisch über Kieselgel 60 (Laufmittel: Dichlormethan/Methanol 100/1 → 100/2) vorgereinigt und nach der Feinreinigung über die präparative RP-HPLC (Methode 5) werden 1.8 g Produkt erhalten.

HPLC (Methode 10): Rₜ = 4.34 min,

MS (DCI (NH₃)) = 366 (M+H)⁺.

¹H-NMR (300 MHz, CDCl₃) : δ = 1.41 (t, 3H), 4.15 (s, 3H), 4.41 (q, 2H), 5.23 (q, 2H), 8.11 (dd, 1H), 8.33 (s, 1H).

Die in der folgenden Tabelle aufgeführten Beispiele 16A bis 19A werden analog zu Beispiel 15A hergestellt.

| **Beispiel-Nr.** | **Struktur** | **Edukt** | **Analytikdaten** |
|---|---|---|---|
| | | **Beispiel-Nr.** | **LC-MS (Methode)/ Meßwerte** |
| **16A** (S)-Enantiomer | 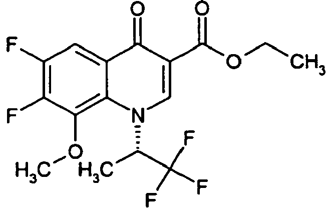 | 11A | LC-MS (Methode 1) : Rₜ = 2.22 min MS (ES+): m/z = 380 (M+H)⁺ |
| **17A** | 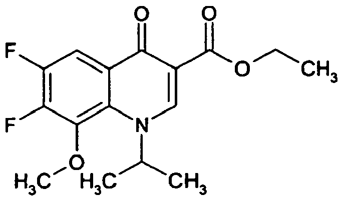 | 12A | LC-MS (Methode 1): Rₜ = 2.16 min MS (ES+): m/z = 326 (M+H)⁺ |
| **18A** | 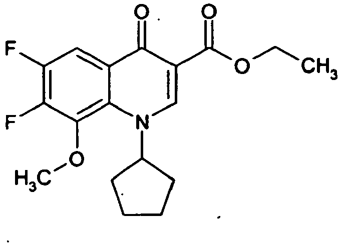 | 13A | LC-MS (Methode 3): Rₜ = 2.46 min MS (ES+): m/z = 352 (m+H)⁺ |
| **19A** (1S,2R)-Enantiomer | 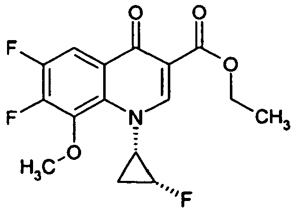 | 14A | LC-MS (Methode 2): Rₜ = 1.76 min MS (ES+): m/z = 342 (M+H)⁺ |

### Beispiel 20A

### 6,7-Difluor-8-methoxy-4-oxo-1-(2,2,2-trifluorethyl)-1,4-dihydrochinolin-3-carbonsäure

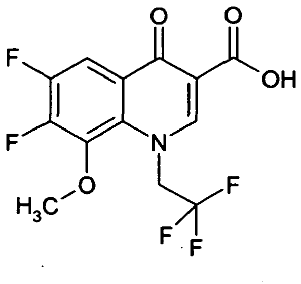

800 mg (2.19 mmol) der Verbindung aus Beispiel 15A werden in einer Mischung aus 25 ml Essigsäure-Wasser-Schwefelsäure 12:8:1 vorgelegt und über Nacht unter Rückfluss gerührt. Zur Aufarbeitung wird das Lösungsmittel am Rotationsverdampfer größtenteils entfernt, der Rückstand unter Eiskühlung vorsichtig mit gesättigter Natriumhydrogencarbonat-Lösung auf pH 3 gestellt, die Suspension mit Wasser verdünnt, der Niederschlag abgesaugt und nach dem Trocknen des Filterrückstandes im Hochvakuum werden 575 mg der Titelverbindung erhalten.

LC-MS (Methode 3): Rₜ = 2.41 min, MS (ES+) = 338 (M+H)⁺.

¹H-NMR (300 MHz, CDCl₃) : δ = 4.21 (s, 3H), 5.37 (q, 2H), 8.11 (dd, 1H), 8.62 (s, 1H), 14.05 (bs, 1H).

Die folgenden Beispiele 21A bis 24A werden analog zu Beispiel 20A hergestellt.

| **Beispiel-Nr.** | **Struktur** | **Edukt** | **Analytikdaten** |
|---|---|---|---|
| | | | **LC-MS (Methode) / Meßwerte HPLC (Methode)/ Meßwert MS (Methode)/ Meßwert** |
| **21A** (S)-Enantiomer | 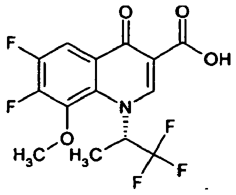 | 16A | HPLC (Methode 10): Rₜ = 4.54 min MS (ESI+): m/z = 374 (M+Na)⁺ |
| **22A** | 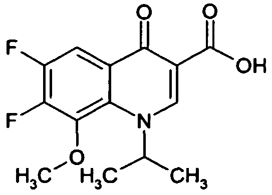 | 17A | LC-MS (Methode 3): Rₜ = 2.27 min MS (ES+): m/z = 298 (M+H)⁺ |
| **23A** | 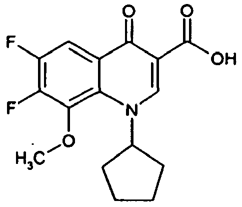 | 18A | LC-MS (Methode 1): Rₜ = 2.40 min MS (ES+): m/z = 324 (M+H)⁺ |
| **24A** (1S,2R)-Enantiomer | 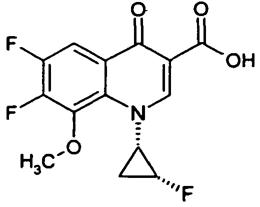 | 19A | LC-MS (Methode 2): Rₜ = 1.84 min MS (ES+): m/z = 313 (M+H)⁺ |

### Beispiel 25A

### [6,7-Difluor-8-methoxy-4-oxo-1-(2,2,2-trifluorethyl)-1,4-dihydrochinolin-3-yl]carbonyl-difluorborat

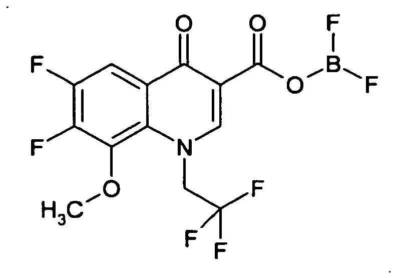

1.5 g (4.30 mmol) der Verbindung aus Beispiel 20A werden in 10 ml Tetrahydrofuran vorgelegt, anschließend mit 6.81 ml (7.63 g, 53.75 mmol) Bortrifluorid-Diethylether-Komplex versetzt und über Nacht bei 70°C nachgerührt. Zur Aufarbeitung wird das auf Raumtemperatur abgekühlte Reaktionsgemisch mit 50 ml Diethylether versetzt, 20 min verrührt und der ausfallende Niederschlag wird abgesaugt. Nach dem Trocknen des Rückstandes im Hochvakuum werden 1150 mg der Titelverbindung erhalten und ohne Reinigung weiter umgesetzt.

HPLC (Methode 9): Rₜ = 4.25 min,

MS (DCI (NH₃)) = 402 (M+NH₄)⁺.

¹H-NMR (300 MHz, DMSO-d₆): δ = 4.21 (s, 3H), 6.12 (q, 2H), 8.38 (dd, 1H), 9.66 (s, 1H).

Die folgenden Beispiele 26A bis 29A werden analog zu Beispiel 25A hergestellt.

| **Beispiel-Nr.** | **Struktur** | **Edukt** | **Analytikdaten** |
|---|---|---|---|
| | | | **LC-MS (Methode)/ Meßwerte** |
| **26A** (S)-Enantiomer | 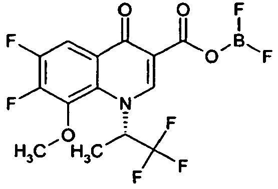 | 21A | LC-MS (Methode 2): Rₜ = 1.98 min MS (ES+): m/z = 400 (M+H)⁺ |
| **27A** | 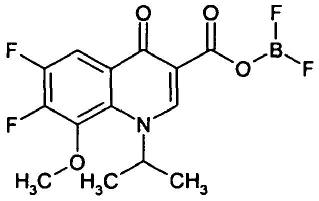 | 22A | LC-MS (Methode 3): Rₜ = 1.83 min MS (ES+): m/z = 346 (M+H)⁺ |
| **28A** | 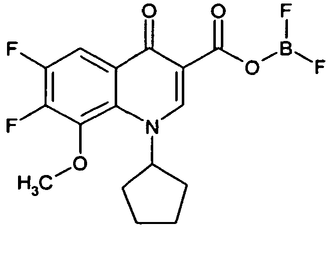 | 23A | LC-MS (Methode 2): Rₜ = 2.02 min MS (ES+): m/z = 372 (M+H)⁺ |
| **29A** (1S,2R)-Enantiomer | 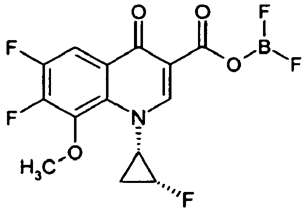 | 24A | LC-MS (Methode 2): Rₜ = 1.74 min MS (ES+): m/z = 361 (M+H)⁺ |

### Beispiel 30A

### [1-Cyclopropyl-7-fluor-8-trifluormethyl-4-oxo-1,4-dihydrochinolin-3-yl]carbonyl-difluorborat

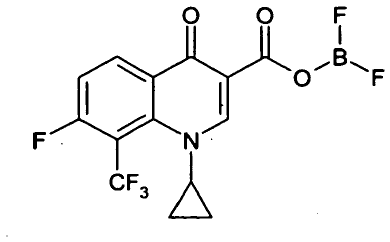

Die Verbindung wird analog zu Beispiel 25A aus 1-Cyclopropyl-7-fluor-8-trifluormethyl-4-oxo-1,4-dihydrochinolin-3-yl]carbonsäure (Herstellung: siehe DE 4301246) hergestellt.

LC-MS (Methode 1): Rₜ = 2.13 min

MS (ES+): m/z = 364 (M+H)⁺

### Beispiel 31A

### 7-[(3RS,5SR)-3,5-Dimethylpiperazin-1-yl]-6-fluor-8-methoxy-4-oxo-1-(2,2,2-trifluorethyl)-1,4-dihydrochinolin-3-carbonsäure - Hydroformiat

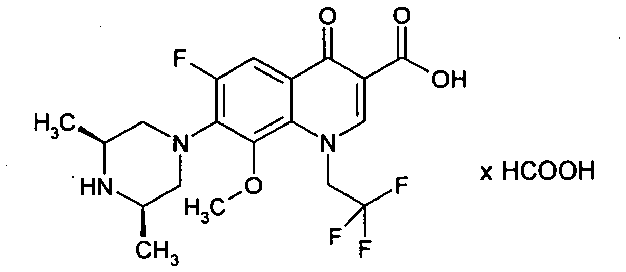

300.0 mg (0.78 mmol) der Verbindung aus Beispiel 25A und 213.6 mg (1.87 mmol) cis-2,6-Dimethylpiperazin werden in 6 ml Acetonitril über Nacht bei 50°C gerührt. Das Lösungsmittel wird vollständig am Rotationsverdampfer entfernt, der Rückstand wird mit einer Mischung aus 12 ml Ethanol und 6 ml Triethylamin 1 h unter Rückfluss gerührt. Zur Aufarbeitung wird das Lösungsmittel am Rotationsverdampfer abgetrennt. Nach der Feinreinigung über die präparative RP-HPLC (Methode 5) werden 260 mg der Zielverbindung erhalten.

HPLC (Methode 9): Rₜ = 3.76 min,

MS (ESI+) = 432 (M+H)⁺.

¹H-NMR (300 MHz, DMSO-d₆) : δ = 1.03 (d, 6H), 2.82 (m, 2H), 3.04 (m, 2H), 3.28 (m, 2H), 3.78 (s, 3H), 5.77 (q, 2H), 7.82 (d, 1H), 8.19 (s, 1H), 8.52 (s, 1H).

Die folgenden Beispiele 32A bis 36A werden analog zu Beispiel 31A hergestellt.

| **Beispiel-Nr.** | **Struktur** | **Edukt** | **Analytikdaten** |
|---|---|---|---|
| | | | **LC-MS (Methode)/ Meßwerte HPLC (Methode)/ Meßwert MS (Methode)/ Meßwert** |
| **32A** (S)-Enantiomer | 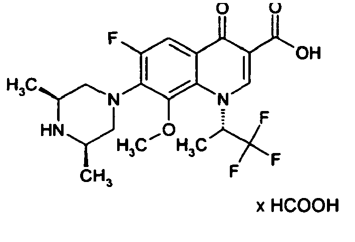 | 26A | HPLC (Methode 10): Rₜ = 3.76 min MS (ESI+): m/z = 446 (M+H)⁺ |
| **33A** | 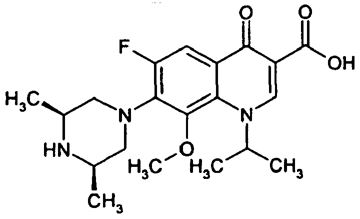 | 27A | HPLC (Methode 9): Rₜ = 3.54 min MS (ESI+): m/z = 392 (M+H)⁺ |
| **34A** | 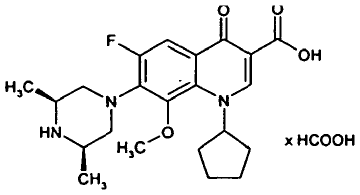 | 28A | HPLC (Methode 9): Rₜ = 3.84 min MS (DCI (NH₃)) : m/z = 418 (M+H)⁺ |
| **35A** (1S,2R)-Enantiomer | 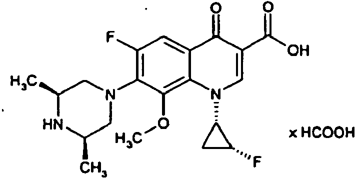 | 29A | LC-MS (Methode 2): Rₜ = 1.02 min MS (ES+): m/z = 407 (M+H)⁺ |
| **36A** | 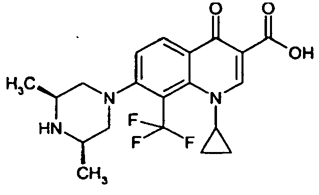 | 30A | LC-MS (Methode 2): Rₜ = 1.08 min MS (ES+): m/z = 410 (M+H)⁺ |

### Beispiel 37A

### N-(2,4-Dichlorbenzyl)-7-[(3RS, 5RS)-3,5-dimethyl-piperazin-1-yl]-6-fluor-8-methoxy-1-(2,2,2-trifluorethyl)-4-oxo-1,4-dihydrochinolin-3-carbonsäureamid

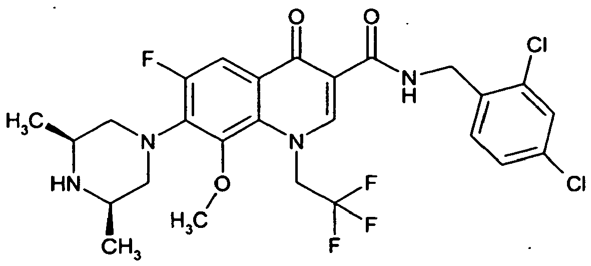

5.20 g (10.58 mmol) 7-(cis-3,5-Dimethylpiperazin-1-yl)-6-fluor-8-methoxy-1-(2,2,2-trifluorethyl)-4-oxo-1,4-dihydrochinolin-3-carbonsäure-Hydroformiat (Beispiel 31A) und 3.73 g (21.2 mmol) 2,4-Dichlorbenzylamin in 10.7 ml Dimethylformamid werden unter Argon mit 7.52 g (58.2 mmol) N,N-Diisopropylethylamin und 7.71 g (14.81 mmol) PyBOP versetzt und über Nacht bei Raumtemperatur gerührt. Das Reaktionsgemisch wird mit 1500 ml Essigsäureethylester verdünnt und dreimal mit Wasser gewaschen. Die vereinigten wässrigen Phasen werden einmal mit Essigsäureethylester extrahiert. Die vereinigten organischen Phasen werden mit gesättigter Natriumchlorid-Lösung gewaschen, über Natriumsulfat getrocknet und eingeengt. Der Rückstand wird mit 30 ml Acetonitril verrührt. Der Feststoff wird dann abfiltriert und im Hochvakuum getrocknet. Man erhält 6.80 mg der Zielverbindung als Cokristallisat mit 1 Äq. Acetonitril. Für kleinere Mengen kann man nach der Extraktion den Rückstand über präparative HPLC reinigen (nach einer der Methoden 5 bis 8).

HPLC (Methode 9): Rₜ = 4.59 min.

MS (ESI) = 589 (M+H)⁺.

¹H-NMR (300 MHz, CDCl₃): δ = 1.11 (d, 6H), 2.01 (3H, CH₃CN), 2.82 (br.t, 2H), 3.03-3.16 (m, 2H), 3.29 (br.d, 2H), 3.79 (s, 3H), 4.69 (d, 2H), 5.25 (q, 2H), 7.20 (dd, 1H), 7.37-7.42 (m, 2H), 7.91 (d, 1H), 8.54 (s, 1H), 10.24 (t, 1H).

### Beispiel 38A

### N-(2,4-Dichlorbenzyl)-7-[(3RS,5RS)-3,5-dimethyl-piperazin-1-yl]-6-fluor-8-methoxy-1-(2,2,2-trifluorethyl)-4-oxo-1,4-dihydrochinolin-3-carbonsäureamid - Hydrochlorid

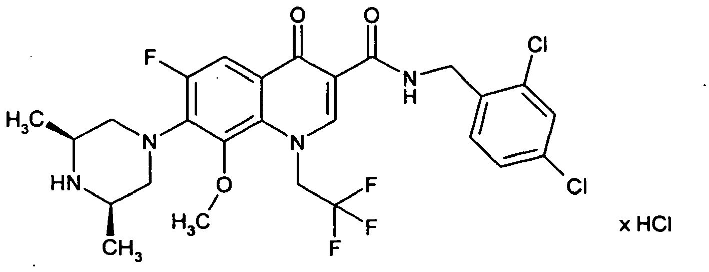

6780 mg der Verbindung aus Beispiel 37A (enthält 1 Äq. Acetonitril) werden in 4.3 ml 4N Chlorwasserstoff in Dioxan gelöst, am Rotationsverdampfer eingeengt und dann im Hochvakuum getrocknet. Ausbeute: 6730 mg (quantitativ).

HPLC (Methode 9): Rₜ = 4.57 min

MS (ESI): m/z = 589 (M+H)⁺

¹H-NMR (400 MHz, DMSO-d₆): δ = 1.38 (d, 6H), 3.222 (br.t, 2H), 3.38-3.52 (m, 4H), 3.83 (s, 3H), 4.60 (d, 2H), 5.71 (q, 2H), 7.39-7.46 (m, 2H), 7.65 (s, 1H), 7.84 (d, 1H), 8.76 (br.s, 1H), 8.89 (s, 1H), 9.35 (br.s, 1H), 10.10 (t, 1H).

### Beispiel 39A

### 1-Cyclopropyl-N-(2,4-dichlorbenzyl)-7-[(3RS,5SR)-3,5-dimethylpiperazin-1-yl]-6-fluor-8-methoxy-4-oxo-1,4-dihydrochinolin-3-carbonsäureamid

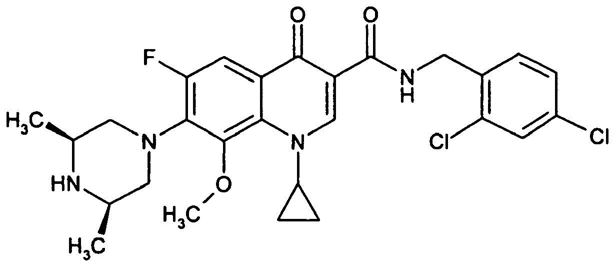

Die Herstellung erfolgt analog zu Beispiel 37A aus 1-Cyclopropyl-7-(cis-3,5-dimethylpiperazin-1-yl)-6-fluor-8-methoxy-4-oxo-1,4-dihydrochinolin-3-carbonsäure (Herstellung siehe: Journal of Medicinal Chemistry, (1995), 38(22), 4478-87).

LC-MS (Methode 2): Rₜ = 1.77 min,

MS (ES+) = 547 (M+H)⁺

Aus der gleichen Säure wie in Beispiel 39A und analog zur Herstellungsvorschrift von Beispiel 37A werden die Beispiele 40A bis 44A hergestellt.

| **Beispiel-Nr.** | **Struktur** | **Amin-Edukt** | **Analytikdaten** |
|---|---|---|---|
| | | **Bsp-Nr.** | **LC-MS (Methode)/ Meßwerte** |
| **40A** | 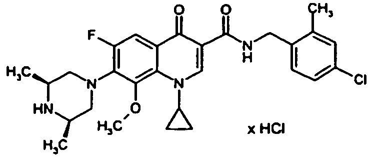 | 4-Chlor-2-methyl-benzylamin | LC-MS (Methode 3): Rₜ = 1.85 min MS (ES+): m/z = 527 (M+H)⁺ |
| **41A** | 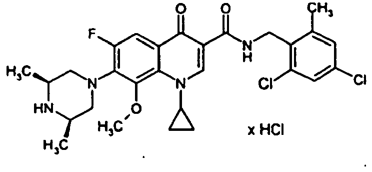 | 8A | LC-MS (Methode 3): Rₜ = 1.91 min MS (ES+): m/z = 561 (M+H)⁺ |
| **42A** | 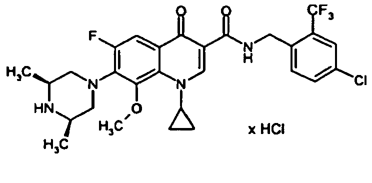 | 9A | LC-MS (Methode 3): Rₜ = 1.91 min MS (ES+): m/z = 581 (M+H)⁺ |
| **43A** | 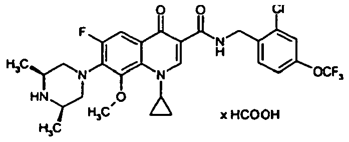 | 7A | LC-MS (Methode 2): Rₜ = 1.86 min MS (ES+): m/z = 597 (M+H)⁺ |
| **44A** | 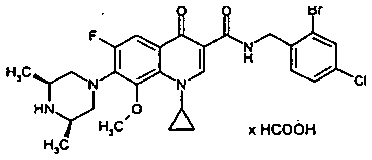 | 6A | LC-MS (Methode 2): Rₜ = 1.74 min MS (ES+): m/z = 591 (M+H)⁺ |

### Beispiel 45A

### 8-Chlor-1-cyclopropyl-N-(2,4-dichlorbenzyl)-7-[(3RS,5SR)-3,5-dimethylpiperazin-1-yl]-6-fluor-4-oxo-1,4-dihydrochinolin-3-carbonsäureamid

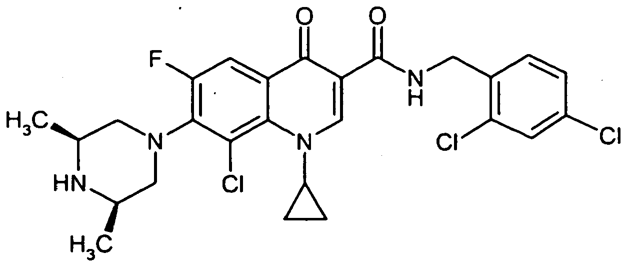

Die Herstellung erfolgt analog zu Beispiel 37A aus 8-Chlor-1-cyclopropyl-7-[(3RS,5SR)-3,5-dimethylpiperazin-1-yl]-6-fluor-4-oxo-1,4-dihydrochinolin-3-carbonsäure (Herstellung siehe: DE 3635218) mit Hydroxybenzotriazol und EDC statt PyBOP.

LC-MS (Methode 2): Rₜ = 1.86 min,

MS (ES+) = 551 (M+H)⁺

¹H-NMR (400 MHz, CDCl₃): δ = 0.9 (m, 2H), 1.1 (d, 6H), 1.2-1.3 (m, 2H), 2.7-2.9 (m, 2H), 3.1-3.3 (m, 4H), 4.3 (m, 1H), 4.7 (d, 2H), 7.2 (dd, 2H), 7.4 (m, 2H), 8.0 (d, 1H), 8.9 (s, 1H), 10.2 (t, 1H).

Aus der gleichen Säure wie in Beispiel 45A und analog zur Herstellungsvorschrift von Beispiel 37A werden die Beispiele 46A und 47A hergestellt.

| **Beispiel-Nr.** | **Struktur** | **Amin-Edukt** | **Analytikdaten** |
|---|---|---|---|
| | | **Bsp-Nr.** | **LC-MS (Methode)/ Meßwerte** |
| **46A** | 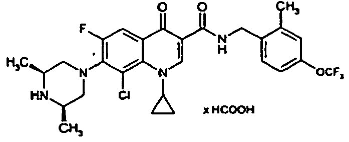 | 5A | LC-MS (Methode 1): Rₜ = 1.91 min MS (ES+): m/z = 581 (M+H)⁺ |
| **47A** | 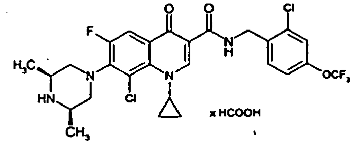 | 7A | LC-MS (Methode 3): Rₜ = 2.02 min MS (ES+): m/z = 601 (M+H)⁺ |

Aus 1-Cyclopropyl-6-fluor-8-methoxy-7-(3-methylpiperazin-1-yl)-4-oxo-1,4-dihydrochinolin-3-carbonsäure (Herstellung: siehe Journal of Medicinal Chemistry 1995, 38(22) 4478) und analog zur Herstellungsvorschrift von Beispiel 37A werden die Beispiele 48A bis 50A hergestellt.

| **Beispiel-Nr.** | **Struktur** | **Amin-Edukt** | **Analytikdaten** |
|---|---|---|---|
| | | **Bsp-Nr.** | **LC-MS (Methode)/ Meßwerte** |
| **48A** | 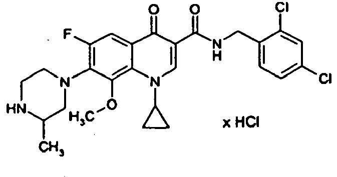 | Dichlorbenzylamin | LC-MS (Methode 2): Rₜ = 2.05min MS (ES+): m/z = 542 (M+H)⁺ |
| **49A** | 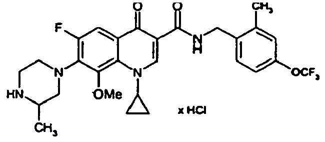 | 5A | LC-MS (Methode 1): Rₜ = 1.86 min MS (ES+): m/z = 563 (M+H)⁺ |
| **50A** | 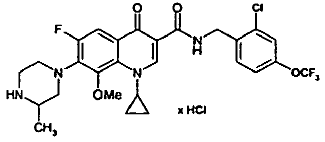 | 7A | LC-MS (Methode 3): Rₜ = 1.98 min MS (ES+): m/z = 583 (M+H)⁺ |

### Beispiel 51A

### N-(4-Brom-2-chlorbenzyl)-7-[(3RS,5RS)-3,5-dimethyl-piperazin-1-yl]-6-fluor-1-(2-fluorethyl)-8-methoxy-4-oxo-1,4-dihydrochinolin-3-carbonsäureamid - Hydroformiat

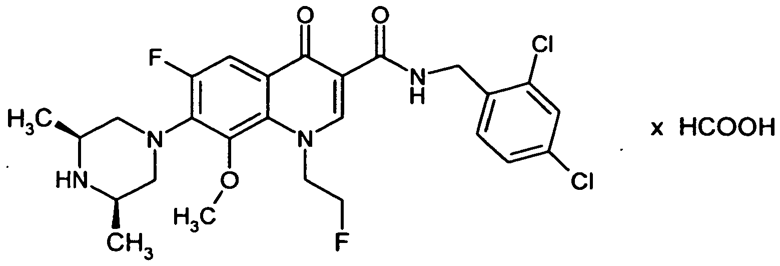

Die Herstellung erfolgt analog zu Beispiel 37A aus 7-(cis-3,5-Dimethylpiperazin-1-yl)-6-fluor-1-(2-fluorethyl)-8-methoxy-4-oxo-1,4-dihydrochinolin-3-carbonsäure (Herstellung siehe: EP 0241206) und 2,4-Dichlorbenzylamin.

HPLC (Methode 9): Rₜ = 4.46 min, MS (ESI) = 553 (M+H)⁺.

¹H-NMR (400 MHz, DMSO-d₆): δ = 1.15 (d, 6H), 2.88-3.07 (m, 2H), 3.11-3.56 (m, 4H unter dem Wassersignal des DMSO), 3.78 (s, 3H), 4.59 (d, 2H), 4.76 (dd, 2H), 4.95 (d, 2H), 7.35-7.50 (m, 2H), 7.64 (s, 1H), 7.83 (d, 1H), 8.16 (s, 1H), 8.72 (s, 1H), 10.27 (t, 1H).

Analog zur Herstellungsvorschrift von Beispiel 37A werden aus verschiedenen Carbonsäuren und Benzylaminen die Beispiele 52A bis 57A hergestellt.

| **Beispiel-Nr.** | **Struktur** | **Edukte** | **Analytikdaten** |
|---|---|---|---|
| | | **Bsp.-Nr.** | **LC-MS (Methode)/ Meßwerte HPLC (Methode)/ Meßwert MS (Methode)/ Meßwert NMR-Spektrum** |
| **52A** (1S,2R)-Enantiomer | 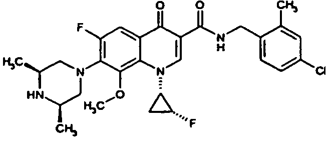 | 35A und 4-Chlor-2-methylbenzylamin | LC-MS (Methode 3): Rₜ = 1.97 min MS (ES+): m/z = 545 (M+H) ^{+ 1}H-NMR (400 MHz, DMSO-d₆): δ = 1.06 (d, 6H), 1.43-1.68 (m, 2H), 2.32 (s, 3H), 2.72-2.91 (m, 2H), 3.06 (m, 2H), 3.25 (m, 2H), 3.77 (s, 3H), 4.08 (m, 1H), 4.51 (d, 2H), 4.93/5.10 (2m, 1H), 7.19-7.32 (m, 3H), 7.71 (d, 1H), 8.68 (s, 1H), 10.08 (t, 1H) |
| **53A** (S)-Enantiomer | 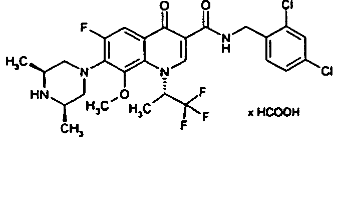 | 32A und 2,4-Dichlorbenzylamin | HPLC (Methode 9): Rₜ = 4.75 min MS (ESI): m/z = 603 (M+ H)⁺ |
| **54A** | 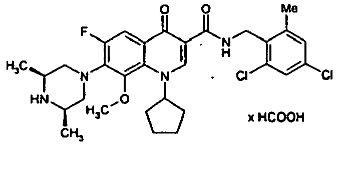 | 34A und 8A | LC-MS (Methode 3): Rₜ = 2.18 min MS (ES+): m/z = 589 (M+H)⁺ |
| **55A** | 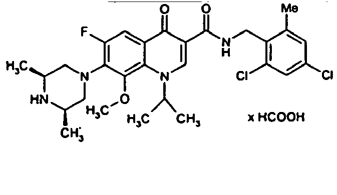 | 33A und 8A | LC-MS (Methode 2): Rₜ = 1.72 min MS (ES+): m/z = 563 (M+H)⁺ |
| **56A** | 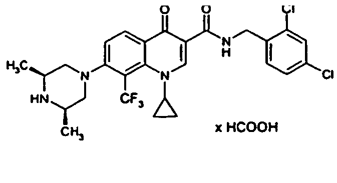 | 36A und 2,4-Dichlorbenzylamin | LC-MS (Methode 3): Rₜ = 2.13 min MS (ES+): m/z = 567 (M+H)⁺ |
| **57A** (1S,2R)-Enantiomer | 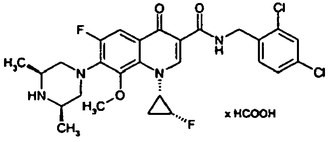 | 35A und 2,4-Dichlorbenzylamin | LC-MS (Methode 1): Rₜ = 1.97 min MS (ES+): m/z = 565 (M+H)⁺ |

### Beispiel 58A

### (3S)-N-(2,4-Dichlorbenzyl)-9,10-difluor-3-methyl-7-oxo-2,3-dihydro-7H-[1,4]oxazino[2,3,4-ij]-chinolin-6-carboxamid

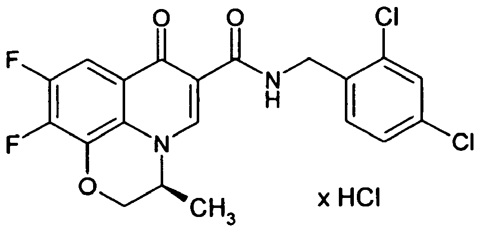

Die Verbindung wird analog zur Vorschrift von Beispiel 37A aus (3S)-9,10-Difluor-3-methyl-7-oxo-2,3-dihydro-7H-[1,4]oxazino[2,3,4-ij]chinolin-6-carbonsäure (Herstellung: siehe Journal of Medicinal Chemistry 1987, 30(12), 2283-2286) und 2,4-Dichlorbenzylamin hergestellt.

LC-MS (Methode 1): Rₜ = 2.59 min

MS (ES+): m/z = 439 (M+H)⁺

¹H-NMR (300 MHz, CDCl₃): δ = 1.62 (d, 3H), 4.38-4.54 (m, 3H), 4.71 (dd, 2H), 7.11 (dd, 1H), 7.49 (d, 1H), 7.50 (d, 1H), 7.86 (dd, 1H), 8.71 (1H), 10.47 (t, 1H).

### Beispiel 59A

### (3S)-N-(2,4-Dichlorbenzyl)-10-{(3RS,5SR)-3,5-dimethylpiperazin-1-yl}-9-fluor-3-methyl-7-oxo-2,3-dihydro-7H-[1,4]oxazino[2,3,4-ij]chinolin-6-carboxamid - Hydrochlorid

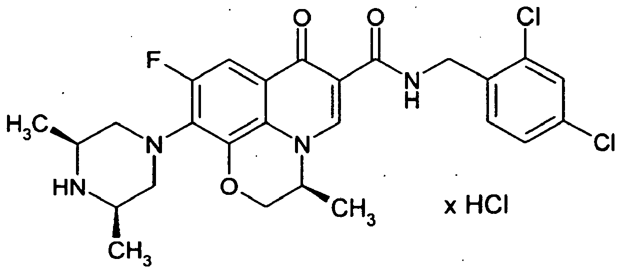

100 mg der Verbindung aus Beispiel 58A werden in 2 ml DMSO mit 39 mg cis-Dimethylpiperazin und 69 mg Triethylamin über Nacht auf 100°C erhitzt und anschließend weitere 15 min. auf 150°C. Nach Abkühlung wird die Reaktionsmischung direkt durch präparative HPLC (Methode 6) getrennt. Man erhält 54 mg (41% d. Th.) der Titelverbindung.

LC-MS (Methode 2): Rₜ = 1.66 min

MS (ES+): m/z = 533 (M+H)⁺.

### Beispiel 60A

### (3RS,5SR)-4-(2-Ethoxy-2-oxoethyl)-3,5-dimethylpiperazin-1-carbonsäure-tert-butylester

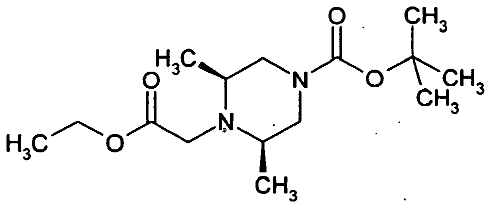

2.0 g (9.33 mmol) (3RS, 5SR)-3,5-Dimethylpiperazin-1-carbonsäure-tert-butylester (Herstellung: Helvetica Chimica Acta 1990, 73 (4), 839-855), 2.3 g (18.67 mmol) Chloressigsäureethylester, 3.9 g (28.00 mmol) Kaliumcarbonat und 0.5 g (2.80 mmol) Kaliumiodid werden in 80 ml Acetonitril über Nacht unter Rückfluss gerührt. Zur Aufarbeitung wird das Acetonitril teilweise am Rotationsverdampfer eingeengt, mit Essigsäureethylester verdünnt, anschließend zweimal mit Wasser und einmal mit gesättigter Natriumchlorid-Lösung gewaschen. Die vereinigten wässrigen Phasen werden einmal mit Essigsäureethylester extrahiert, die vereinigten organischen Phasen über Natriumsulfat getrocknet und das Lösungsmittel wird vollständig am Rotationsverdampfer entfernt. Der Rückstand wird mit Cyclohexan aufgenommen und nach anschließender Eluierung über Kieselgel 60 mittels Unterdruck (Cyclohexan/Essigsäureethylester 100/10 → 80/10 → 40/10) werden 2.5 g (88% d. Th.) Produkt erhalten.

MS (ESI+): m/z = 301 (M+H)⁺

¹H-NMR (300 MHz, CDCl₃): δ = 1.08 (d, 6H), 1.28 (t, 3H), 1.45 (s, 9H), 2.53 (m, 2H), 2.89 (m, 2H), 3.57 (s, 2H), 3.70-4.06 (m, 2H), 4.17 (q, 2H).

### Beispiel 61A

### (3RS,5SR)-4-(2-Ethoxy-1-methyl-2-oxoethyl)-3,5-dimethylpiperazin-1-carbonsäure-tert-butylester

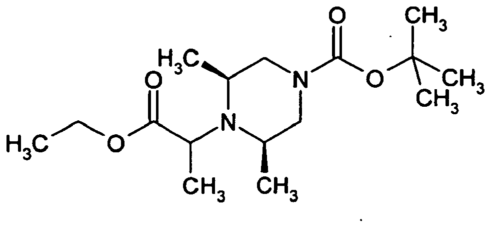

0.5 ml (377.2 mg, 3.73 mmol) Diisopropylamin werden in 2 ml Tetrahydrofuran bei -10°C vorgelegt, langsam mit 2.3 ml (238.8 mg, 3.73 mmol) 1.6 M n-Butyllithium-Lösung in Tetrahydrofuran versetzt und es wird nach erfolgter Zugabe für 10 min bei 5°C nachgerührt. 400.0 mg (1.33 mmol) der Verbindung aus Beispiel 60A werden in einer Mischung aus 4 ml Tetrahydrofuran und 1 ml Hexamethylphosphorsäuretriamid bei -78°C vorgelegt, langsam die Hälfte der frisch hergestellten Lithiumdiisopropylamid-Lösung zugegeben, 45 min bei -78°C nachgerührt, die Reaktion auf -15°C erwärmt und bei dieser Temperatur 15 min nachgerührt. Die Mischung wird nochmals auf -78°C abgekühlt, anschließend 0.1 ml (245.7 mg, 1.73 mmol) Iodmethan, gelöst in 1 ml Tetrahydrofuran, zugetropft und die Reaktion unter Rühren auf Raumtemperatur über Nacht erwärmt. Es wird nochmals auf -78°C abgekühlt, mit der restlichen Lithiumdiisopropylamid-Lösung versetzt, 45 min bei -78°C nachgerührt, 20 min bei -15°C gerührt, nochmals mit 0.1 ml (245.7 mg, 1.73 mmol) Iodmethan bei -78°C tropfenweise versetzt und über Nacht wird die Mischung langsam unter Rühren auf Raumtemperatur gebracht. Zur Aufarbeitung wird die Reaktion mit 10%iger Ammoniumchlorid-Lösung gequencht, mit Essigsäureethylester verdünnt, anschließend nacheinander einmal mit gesättigter Natriumhydrogencarbonat-Lösung, zweimal mit Wasser und einmal mit gesättigter Natriumchlorid-Lösung gewaschen, die organische Phase über Natriumsulfat getrocknet, filtriert und das Lösungsmittel am Rotationsverdampfer vollständig entfernt. Der Rückstand wird zur Feinreinigung über Kieselgel 60 mittels Unterdruck (Eluent: Cyclohexan/Essigsäureethylester 40/10) chromatographiert und es werden 265 mg (58% d. Th.) Produkt erhalten.

HPLC (Methode 10): Rₜ = 3.70 min;

MS (ESI+): m/z = 315 (M+H)⁺

¹H-NMR (300 MHz, CDCl₃) δ = 1.01 (d, 3H), 1.09 (d, 3H), 1.27 (m, 6H), 1.45 (s, 9H), 2.78-2.95 (m, 3H), 3.03 (m, 1H), 3.53-3.83 (m, 3H: darin 3.78 (q, 1H)), 4.18 (q, 2H).

### Beispiel 62A

### (3RS,5SR)-4-(2-Ethoxy-1,1-dimethyl-2-oxoethyl)-3,5-dimethylpiperazin-1-carbonsäure-tert-butylester

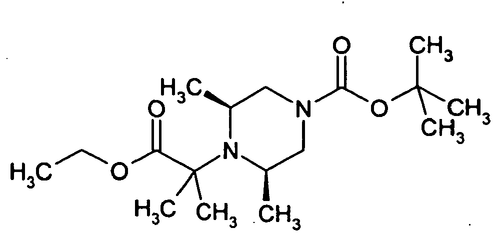

Aus dem Experiment beschrieben unter Beispiel 61A erhält man als zweites Produkt 70 mg der Titelverbindung.

HPLC (Methode 10): Rₜ = 4.02 min;

MS (ESI+): m/z = 329 (M+H)⁺

### Beispiel 63A

### 2-[(2RS,6SR)-2,6-Dimethylpiperazin-1-yl]propansäureethylester - Bistrifluoracetat

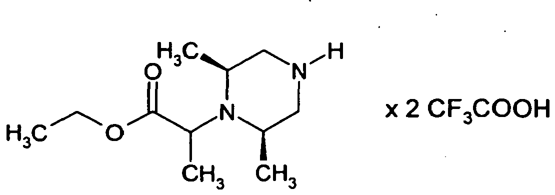

120.0 mg (0.38 mmol) der Verbindung aus Beispiel 61A werden in 2 ml einer Mischung aus Trifluoressigsäure/Dichlormethan (1/1) gelöst und 20 min bei Raumtemperatur nachgerührt. Zur Aufarbeitung wird das Lösungsmittel vollständig am Rotationsverdampfer entfernt und nach dem Trocknen im Hochvakuum werden 155 mg (92% d. Th.) Produkt erhalten.

MS (ESI+): m/z = 214 (M+H)⁺;

¹H-NMR (400 MHz, CDCl₃) : δ = 1.31-1.43 (m, 6H), 1.57 (d, 3H), 1.69 (d, 3H), 3.55 (m, 2H), 3.92-4.48 (m, 7H).

### Beispiel 64A

### 2-[(2RS,6SR)-2,6-Dimethylpiperazin-1-yl]-2-methylpropansäureethylester - Bistrifluoracetat

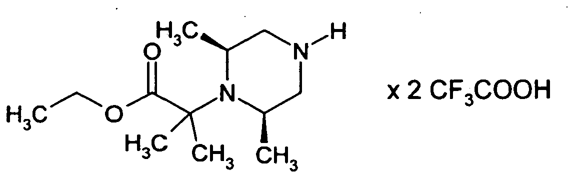

Analog zu Beispiel 63A wird aus Beispiel 62A die Titelverbindung gewonnen. Sie wird direkt weiter zu Beispiel 66A umgesetzt.

### Beispiel 65A

### 1-Cyclopropyl-7-[(3RS,5SR)-4-(2-ethoxy-1-methyl-2-oxoethyl)-3,5-dimethylpiperazin-1-yl]-6-fluor-8-methoxy-4-oxo-1,4-dihydrochinolin-3-carbonsäure

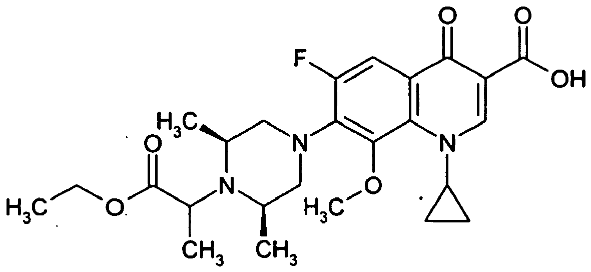

Zur Freisetzung der Base aus der Verbindung aus Beispiel 63A werden 120.2 mg (0.35 mmol) der Verbindung aus Beispiel 63A in 2 ml Dichlormethan/Acetonitril (1/1) vorgelegt, anschließend mit 400 mg Tris-(2-aminoethyl)amin-Polystyrol versetzt, 20 min bei Raumtemperatur nachgerührt, filtriert, der Rückstand mit Dichlormethan gewaschen und das Dichlormethan wird am Rotationsverdampfer bei einem Unterdruck von 500 mbar abdestilliert. Zu dieser Lösung werden 155.0 mg (0.35 mmol) (1-Cyclopropyl-6-7-difluor-8-methoxy-4-oxo-1,4-dihydrochinolin-3-yl)-carbonyl-Difluoridoborat (Herstellung: siehe Journal of Medicinal Chemistry 1995, 38(22), 4478-4487) zugegeben und die Reaktion wird über Nacht bei 50°C gerührt. Das Lösungsmittel wird vollständig am Rotationsverdampfer entfernt, der Rückstand mit 2.5 ml Triethylamin und 5.0 ml Ethanol versetzt, und es wird für 1 h unter Rückfluss gerührt. Die Lösungsmittel werden abrotiert und nach der Feinreinigung des Rückstandes über die präparative RP-HPLC (Methode 5) werden 45 mg (26% d. Th.) Produkt erhalten.

HPLC (Methode 9): Rₜ = 3.85 min;

MS (ESI+): m/z = 490 (M+H)⁺;

¹H-NMR (400 MHz, CDCl₃): δ = 0.93-1.10 (m, 5H: darin 1.08 (d, 3H)), 1.12-1.24 (m, 5H: darin 1.18 (d, 3H)), 1.28-1.39 (m, 6H), 3.10 (m, 2H), 3.21 (m, 2H), 3.35 (m, 1H), 3.49 (m, 1H), 3.73 (s, 3H), 3.89 (q, 1H), 4.01 (m, 1H), 4.22 (q, 2H), 7.87 (d, 1H), 8.81 (s, 1H).

### Beispiel 66A

### 1-Cyclopropyl-7-[(3RS,5SR)-4-(2-ethoxy-1,1-dimethyl-2-oxoethyl)-3,5-dimethylpiperazin-1-yl]-6-fluor-8-methoxy-4-oxo-1,4,dihydrochinolin-3-carbonsäure

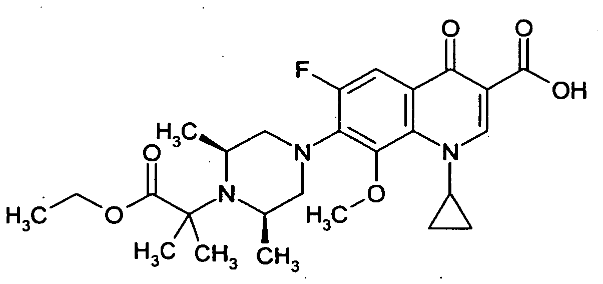

Analog zu Beispiel 65A wird aus der Verbindung aus Beispiel 64A die Titelverbindung hergestellt.

LC-MS (Methode 1): Rₜ = 2.13 min;

MS (ES+): m/z = 504 (M+H)⁺.

### Ausführungsbeispiele

### Allgemeine Arbeitsvorschrift 1: Alkylierung der Piperazinderivate

Das Piperazinderivat (1 Äq.), 2.5 Äq. Alkylierungsmittel, 4.5 Äq. Kaliumcarbonat und 0.3 Äquivalente Kaliumiodid werden in Acetonitril über Nacht unter Rückfluss gerührt. Der abgekühlte Ansatz wird mit Dichlormethan verdünnt, anschließend zweimal mit Wasser gewaschen, die organische Phase über Natriumsulfat getrocknet, filtriert und das Lösungsmittel am Rotationsverdampfer vollständig entfernt. Aus dem Rückstand wird nach der Trennung über die präparative RP-HPLC die Zielverbindung erhalten. Als Alkylierungsmittel werden Chloressigsäureethylester, Bromessigsäureethylester oder 2-Brompropansäureethylester eingesetzt.

### Beispiel 1

### {(2RS,6SR)-4-[3-{[(2,4-Dichlorbenzyl)amino]carbonyl}-6-fluor-8-methoxy-4-oxo-1-(2,2,2-trifluorethyl)-1,4-dihydrochinolin-7-yl]-2,6-dimethylpiperazin-1-yl}ethansäureethylester - Hydroformiat

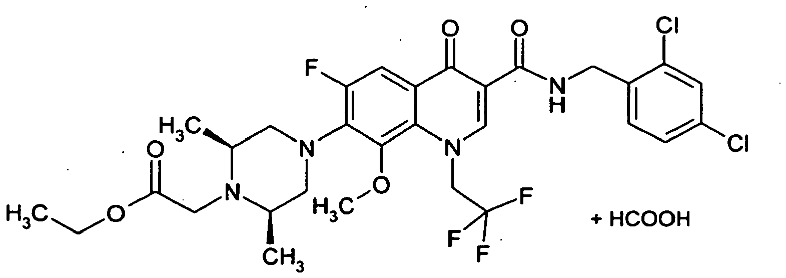

810.0 mg (1.29 mmol) N-(2,4-Dichlorbenzyl)-7-[(3RS,5SR)-3,5-dimethylpiperazin-1-yl]-6-fluor-8-methoxy-4-oxo-1-(2,2,2-trifluorethyl)-1,4-dihydrochinolin-3-carbonsäureamid - Hydrochlorid (Beispiel 38A), 396.5 mg (3.24 mmol) Chloressigsäureethylester, 64.5 mg (0.39 mmol) Kaliumiodid und 804.9 mg (5.82 mmol) Kaliumcarbonat werden in 20 ml Acetonitril über Nacht unter Rückfluss gerührt. Die Aufarbeitung erfolgt analog der allgemeinen Arbeitsvorschrift 1 und nach der Reinigung über die präparative RP-HPLC (Methode 5) werden 1050 mg der Zielverbindung erhalten.

HPLC (Methode 9): Rₜ = 4.87 min,

MS (ESI+) = 675 (M+H)⁺

¹H-NMR (300 MHz, CDCl₃): δ = 1.15 (d, 6H), 1.41 (t, 3H), 2.99-3.14 (m, 2H), 3.18-3.37 (m, 4H), 3.66 (s, 2H), 3.83 (s, 3H), 4.19 (q, 2H), 4.69 (d, 2H), 5.26 (q, 2H), 7.21 (dd, 1H), 7.38 (m, 2H); 7.90 (d, 1H), 8.03 (s, 1H), 8.58 (s, 1H), 10.28 (t, 1H).

### Beispiel 2

### ((2RS,6SR)-4-{3-{[(2,4-Dichlorbenzyl)amino]carbonyl}-6-fluor-1-[(1S,2R)-2-fluorcyclopropyl]-8-methoxy-4-oxo-1,4-dihydrochinolin-7-yl}-2,6-dimethylpiperazin-1-yl)ethansäureethylester

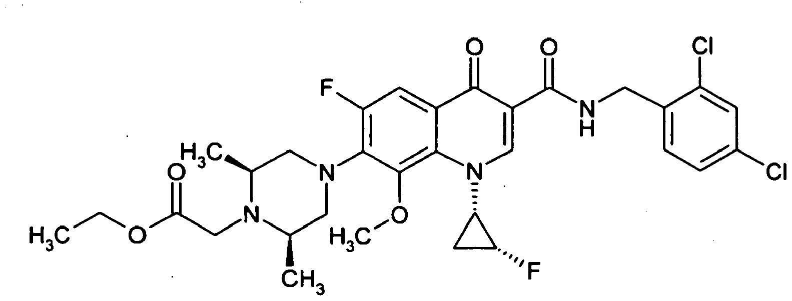

Aus 40 mg (0.05 mmol) N-(2,4-Dichlorbenzyl)-7-[(3RS,5SR)-3,5-dimethylpiperazin-1-yl]-6-fluor-1-[(1S,2R)-2-fluorcyclopropyl]-8-methoxy-4-oxo-1,4-dihydrochinolin-3-carbonsäureamid (Beispiel 57A), 7 µl (11.1 mg, 0.07 mmol) Bromessigsäureethylester, 17.3 mg (0.10 mmol) Kaliumiodid, 8 µl (6.1 mg, 0.05 mmol) N,N-Diisopropylethylamin und 16.4 mg (0.12 mmol) Kaliumcarbonat werden, wie in der allgemeinen Arbeitsvorschrift 1 zur Alkylierung der Piperazinderivate beschrieben wird, ohne weitere Reinigungsverfahren 10 mg vom Zielprodukt erhalten.

LC-MS (Methode 3): Rₜ = 2.41 min,

MS (ES+) = 651 (M+H)⁺

Nach der allgemeinen Arbeitsvorschrift 1 werden aus den angegebenen Edukten die folgenden Beispiele 3 bis 8 hergestellt.

| **Beispiel-Nr.** | **Struktur** | **Edukte** | **Analytikdaten** |
|---|---|---|---|
| | | **Bsp.-Nr.** | **LC-MS (Methode)/ Meßwerte HPLC (Methode)/ Meßwert MS (Methode)/ Meßwert NMR-Spektrum** |
| 3 | 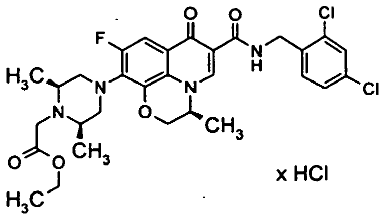 | 59A | LC-MS (Methode 3): Rₜ = 2.19 min MS (ES+): m/z = 619 (M+H)⁺ |
| 4 | 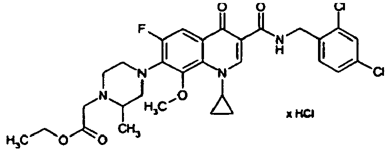 | 48A | LC-MS (Methode 1): Rₜ = 2.21 min MS (ES+): m/z = 619 (M+H)⁺ |
| 5 | 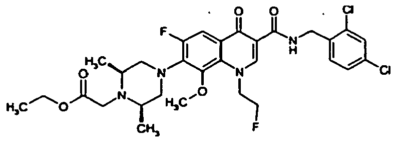 | 51A | HPLC (Methode 9): **Rₜ** = 4.66 min MS (ESI): m/z = 639 (M+H)⁺ |
| 6 | 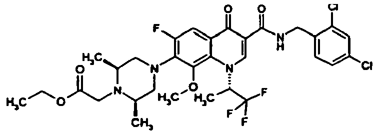 | 53A | HPLC (Methode 9): Rₜ = 4.93 min MS (ESI): m/z = 689 (M+H)⁺ |
| 7 | 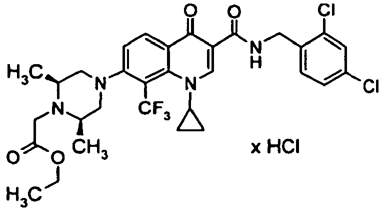 | 56A | LC-MS (Methode 2): Rₜ = 2.78 min MS (ES+): m/z = 653 (M+H)⁺ |
| 8 | 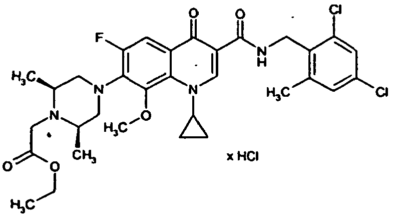 | 41A | LC-MS (Methode 3): Rₜ= 2.56 min MS (ES+): m/z = 647 (M+H)⁺ |

### Beispiel 9

### 4-[3-{1-Cyclopropyl-[(2,4-dichlorbenzyl)amino]carbonyl}-1-fluor-8-methoxy-4-oxo-1,4-dihydrochinolin-7-yl]-(2RS,6SR)-2,6-dimethylpiperazin-1-yl}ethansäureethylester - Hydrochlorid

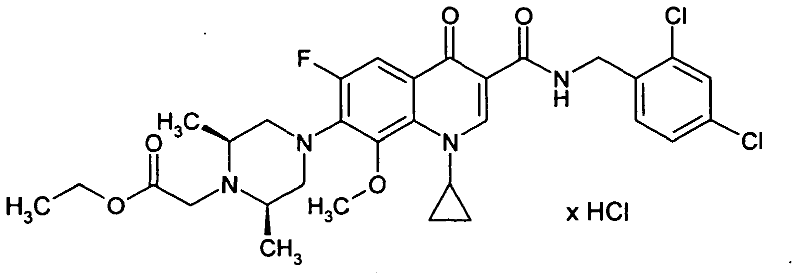

1 g der Verbindung aus Beispiel 39A wird mit 343 mg Ethylbromacetat, 312 mg Kaliumiodid und 590 mg Kaliumcarbonat in 60 ml Acetonitril für 2 h unter Rückfluss erhitzt. Nach Abkühlung wird die Reaktionsmischung über präparative HPLC (Methode 6) getrennt. Man erhält 862 mg (75% d. Th.) der Titelverbindung.

LC-MS (Methode 2): Rₜ = 2.39 min, MS (ESI): m/z = 633 (M+H)⁺.

¹H-NMR (400 MHz, DMSO-d₆): δ = 0.98 (m, 2H), 1.12 (m, 2H), 1.29 (t, 3H), 1.33 (d, 6H), 3.35-3.69 (m, 4H), 3.72-3.90 (m, 5H: darin 3.79 (s, 3H)), 4.11 (m, 1H), 4.23-4.51 (m, 4H: darin 4.29 (q, 2H)), 4.59 (d, 2H), 7.39 (d, 1H), 7.42 (dd, 1H), 7.53 (d, 1H), 7.78 (d, 1H), 8.69 (s, 1H), 10.22 (t, 1H).

Nach der allgemeinen Arbeitsvorschrift 1 werden aus den angegebenen Edukten die folgenden Beispiele 10 bis 22 hergestellt.

| **Beispiel-Nr.** | **Struktur** | **Edukte** | **Analytikdaten** |
|---|---|---|---|
| | | **Bsp.-Nr.** | **LC-MS (Methode)/ Meßwerte HPLC (Methode)/ Meßwert MS (Methode)/ Meßwert NMR-Spektrum** |
| **10** | 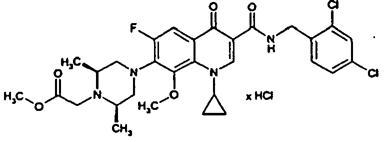 | 39A | LC-MS (Methode 1): Rₜ = 2.31 min MS (ES+): m/z = 619 (M+H)⁺ |
| **11** | 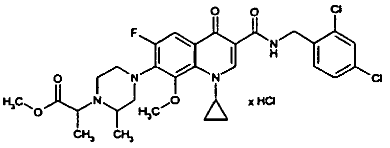 | 48A | LC-MS (Methode 3): Rₜ = 2.16/2.47 min MS (ES+): m/z = 619 (M+H)⁺ |
| **12** | 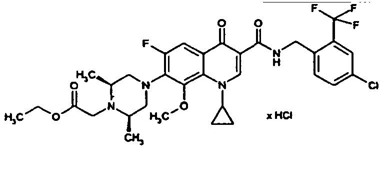 | 42A | LC-MS (Methode 3): Rₜ = 2.55min MS (ES+): m/z = 667 (M+H)⁺ |
| **13** | 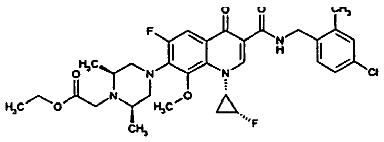 | 52A | LC-MS (Methode 3): Rₜ = 2.35min MS (ES+): m/z = 631 (M+H)⁺ |
| **14** | 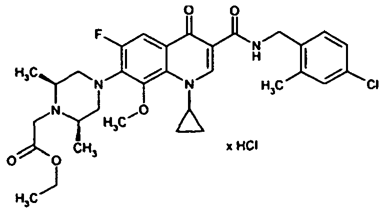 | 40A | LC-MS (Methode 1): = 2.41min MS (ES+): m/z = 613 (M+H)⁺ |
| **15** | 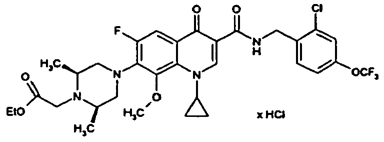 | 43A | LC-MS (Methode 2): Rₜ = 2.46 min MS (ES+): m/z = 683 (M+H)⁺ |
| **16** | 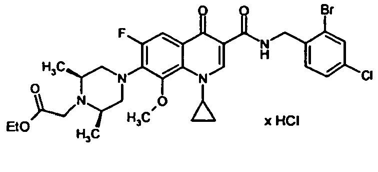 | 44A | LC-MS (Methode 2): Rₜ = 2.38 min MS (ES+): m/z = 677/679 (M+H)⁺ |
| **17** | 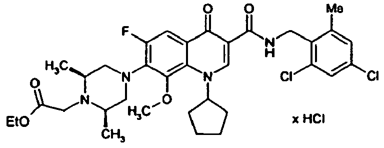 | 54A | LC-MS (Methode 3): Rₜ = 2.91 min MS (ES+): m/z = 675 (M+H)⁺ |
| **18** | 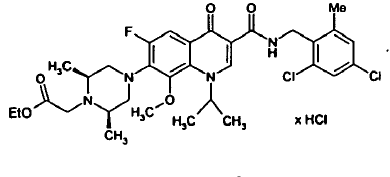 | 55A | LC-MS (Methode 2): Rₜ = 2.54 min MS (ES+): m/z = 649 (M+H)⁺ |
| **19** | 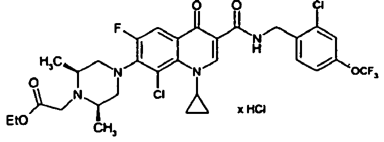 | 47A | LC-MS (Methode 11): Rₜ = 2.58 min MS (ES+): m/z = 687 (M+H)⁺ |
| **20** | 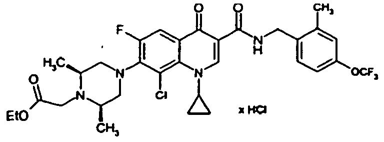 | 46A | LC-MS (Methode 2): Rₜ = 2.52 min MS (ES+): m/z = 667 (M+H)⁺ |
| **21** | 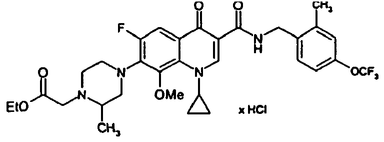 | 49A | LC-MS (Methode 2): Rₜ = 2.27 min MS (ES+): m/z = 649 (M+H)⁺ |
| **22** | 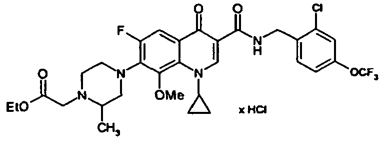 | 50A | LC-MS (Methode 2): Rₜ = 2.32 min MS (ES+): m/z = 669 (M+H)⁺ |

### Beispiel 23

### ((2RS,6SR)-4-[3-{[(2,4-Dichlorbenzyl)amino]carbonyl}-6-fluor-8-methoxy-4-oxo-1-(2,2,2-trifluorethyl)-1,4-dihydrochinolin-7-yl]-2,6-dimethylpiperazin-1-yl}(oxo)ethansäureethylester

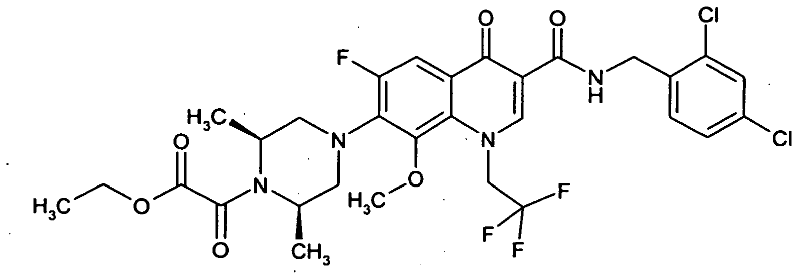

200 mg der Verbindung aus Beispiel 37A werden in 3 ml Dichlormethan und 0.26 ml Pyridin gelöst, dann mit 71 µl Oxalsäureethylesterchlorid versetzt. Man lässt die Mischung 3 h bei RT rühren. Zur Aufarbeitung wird die Mischung mit Essigsäureethylester verdünnt; zweimal mit Wasser, einmal mit gesättigter Natriumhydrogencarbonat-Lösung und einmal mit gesättigter Natriumchlorid-Lösung ausgeschüttelt. Die organische Phase wird über Natriumsulfat getrocknet, in Vakuum eingeengt und im Hochvakuum getrocknet. Man erhält 220 mg der Titelverbindung.

LC-MS (Methode 3): Rₜ = 3.19 min

MS (ES+): m/z = 689 (M+H)⁺.

### Beispiel 24

### 1-Cyclopropyl-N-(2,4-dichlorbenzyl)-7-[(3RS,5SR)-3,5-dimethyl-4-(2-ethoxycarbonyl-ethyl)piperazin-1-yl]-6-fluor-8-methoxy-4-oxo-1,4-dihydrochinolin-3-carbonsäureamid - Hydrochlorid

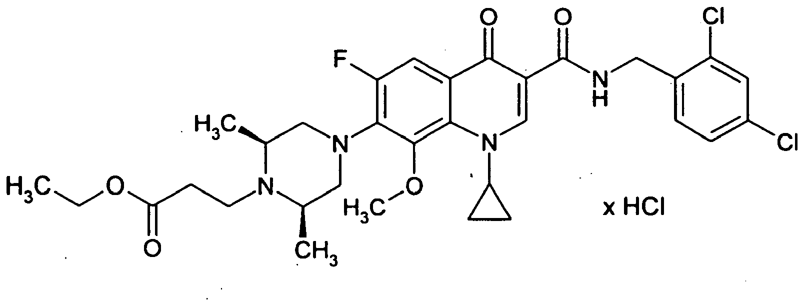

150 mg der Verbindung aus Beispiel 39A werden mit 4 Äq. Lithiumperchlorat vorgelegt und mit Acrylsäureethylester im Überschuss (ca. 400 µl) versetzt. Die Suspension wird 12 h bei RT gerührt und dann direkt über die präparative HPLC (Methode 6) gereinigt. Man erhält 79 mg (42% d. Th.) der Titelverbindung.

LC-MS (Methode 3): Rₜ = 2.09 min

MS (ES+): m/z = 647 (M+H)⁺

¹H-NMR (400 MHz, DMSO-d₆): δ = 0.97 (m, 2H), 1.11 (m, 2H), 1.23 (t, 3H), 1.37 (d, 6H), 2.94 (m, 2H), 3.43-3.68 (m, 8H), 3.71 (s, 3H), 4.09 (m, 1H), 4.14 (q, 2H), 4.58 (d, 2H), 7.38 (d, 1H), 7.42 (dd, 1H), 7.64 (d, 1H), 7.76 (d, 1H), 8.79 (s, 1H), 10.21 (t, 1H).

### Beispiel 25

### N-{[(2RS,6SR)-4-(1-Cyclopropyl-3-{[(2,4-dichlorbenzyl)-amino]carbonyl}-6-fluor-8-methoxy-4-oxo-1,4-dihydrochinolin-7-yl)-2,6-dimethylpiperazin-1-yl]carbonyl}glycinethylester

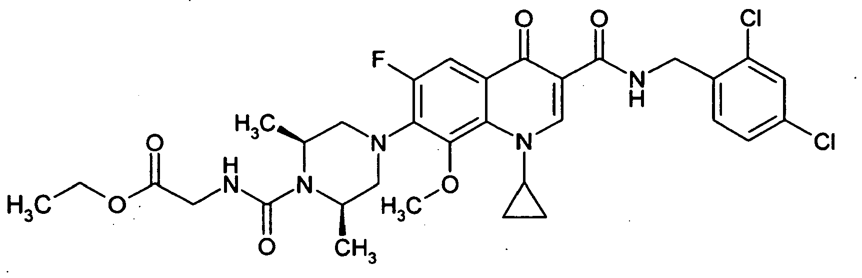

100 mg (0.18 mmol) der Verbindung aus Beispiel 39A werden zu einer Lösung von 28 mg (0.22 mmol) Isocyanatoessigsäureethylester in 5 ml Dichlormethan gegeben. Man läßt die Reaktionsmischung über Nacht bei RT rühren. Nach Entfernung des Lösungsmittels am Rotationsverdampfer wird der Rückstand durch RP-HPLC (Methode 6) gereinigt. Man erhält 73 mg (59% d.Th.) der Titelverbindung.

LC-MS (Methode 3): Rₜ = 2.88 min

MS (ESI): m/z = 676 (M+H)⁺

### Beispiel 26

### N-{[(2RS,6SR)-4-(1-Cyclopropyl-3-{[(2,4-dichlorbenzyl)amino]carbonyl}-6-fluor-8-methoxy-4-oxo-1,4-dihydrochinolin-7-yl)-2,6-dimethylpiperazin-1-yl]carbonyl}alaninsäureethylester

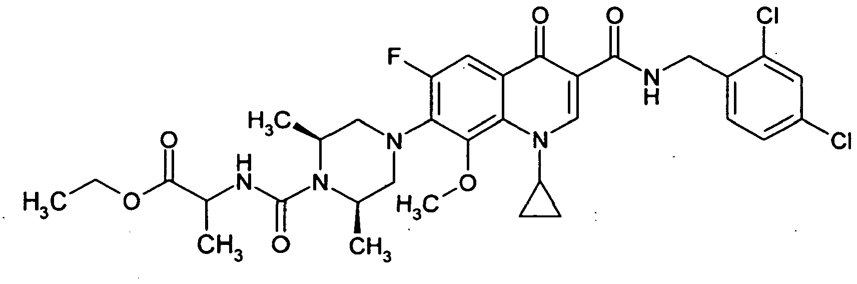

Die Titelverbindung wird analog zu Beispiel 25 aus der Verbindung aus Beispiel 39A und 1-Ethoxycarbonylethylisocyanat hergestellt.

LC-MS (Methode 2): Rₜ = 2.80 min

MS (ESI): m/z = 690 (M+H)⁺

### Beispiel 27

### 2-[(2RS,6SR)-4-(1-Cyclopropyl-3-{[(2,4-dichlorbenzyl)amino]carbonyl}-6-fluor-8-methoxy-4-oxo-1,4-dihydrochinolin-7-yl)-2,6-dimethylpiperazin-1-yl]propansäureethylester

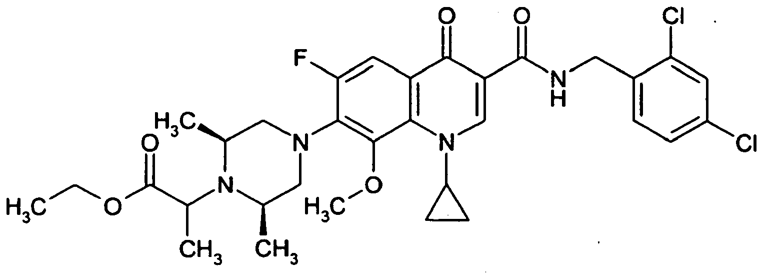

43.0 mg (0.08 mmol) der Verbindung aus Beispiel 65A, 28.3 mg (0.16 mmol) 2,4-Dichlorbenzylamin und 77 µl (57.1 mg, 0.44 mmol) N,N-Diisopropylethylamin werden in 5.2 ml N,N-Dimethylformamid vorgelegt, mit 104.5 mg (0.20 mmol) PyBOP versetzt und der Ansatz wird für 3 h bei Raumtemperatur nachgerührt. Zur Aufarbeitung wird die Reaktionsmischung mit Essigsäureethylester versetzt, zweimal mit Wasser ausgeschüttelt, die vereinigten wässrigen Phasen mit Essigsäureethylester extrahiert, die vereinigten organischen Phasen über Natriumsulfat getrocknet, filtriert und das Lösungsmittel wird am Rotationsverdampfer vollständig abgetrennt. Nach der Feinreinigung des erhaltenen Rückstandes über die präparative RP-HPLC (Methode 5) werden 48 mg (92% d. Th.) Produkt erhalten.

LC-MS (Methode 2): Rₜ = 2.22 min, MS (ES+): m/z = 647 (M+H)⁺;

¹H-NMR (400 MHz, CDCl₃): δ = 0.94 (m, 2H), 1.06 (d, 3H), 1.11-1.21 (m, 5H: darin 1.18 (d, 3H)), 1.28-1.39 (m, 6H), 2.98 (m, 1H), 3.08 (m, 1H), 3.18 (m, 2H), 3.31 (m, 1H), 3.43 (m, 1H), 3.72 (s, 3H), 3.89 (q, 1H), 3.95 (m, 1H), 4.23 (q, 2H), 4.69 (d, 2H), 7.18 (dd, 1H), 7.38 (s, 1H), 7.40 (d, 1H), 7.85 (d, 1H), 8.82 (s, 1H), 10.39 (t, 1H).

### Allgemeine Arbeitsvorschrift 2: Verseifung der Ester zu Carbonsäuren

1.0 Äquivalent Ester und 5.0 Äquivalent 1M Lithiumhydroxid-Lösung werden über Nacht in Dioxan bei Raumtemperatur gerührt. Zur Aufarbeitung wird die Reaktion mit Wasser verdünnt, anschließend mit 1M Salzsäure auf pH 3 gestellt, dreimal mit Dichlormethan extrahiert und die vereinigten organischen Phasen werden über Natriumsulfat getrocknet. Das Natriumsulfat wird abfiltriert und das Lösungsmittel am Rotationsverdampfer vollständig entfernt. Nach der Feinreinigung über die präparative RP-HPLC (Methode 5 oder 6) wird die Zielverbindung erhalten.

### Beispiel 28

### {(2RS,6SR)-4-[3-{[(2,4-Dichlorbenzyl)amino]carbonyl}-6-fluor-8-methoxy-4-oxo-1-(2,2,2-trifluorethyl)-1,4-dihydrochinolin-7-yl]-2,6-dimethylpiperazin-1-yl}ethansäure - Hydroformiat

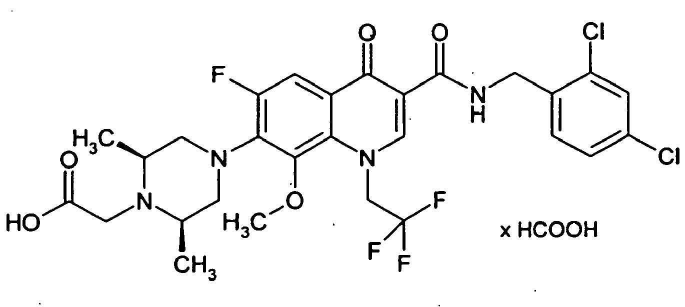

45.0 mg (0.06 mmol) der Verbindung aus Beispiel 1 werden in ml Dioxan gelöst, anschließend mit 0.31 ml (0.31 mmol) 1M Lithiumhydroxid-Lösung versetzt und über Nacht bei Raumtemperatur nachgerührt. Zur Aufarbeitung wird das Lösungsmittel am Rotationsverdampfer entfernt, der Rückstand mit Wasser aufgenommen, mit 1M Salzsäure angesäuert (pH 3) und der ausfallende Niederschlag wird mit wenig Dimethylsulfoxid aufgelöst. Aus der Lösung wird nach der Feinreinigung über die präparative RP-HPLC (Methode 5) die Zielverbindung mit 38 mg erhalten.

HPLC (Methode 9): Rₜ = 4.62 min,

MS (ESI+) = 647 (M+H)⁺.

¹H-NMR (300 MHz, DMSO-d₆): δ = 1.04 (m, 6H), 2.90-3.85 (m, 11H: darin 3.78 (s, 3H)), 4.69 (d, 2H), 5.70 (m, 2H), 7.36-7.48 (m, 2H), 7.65 (d, 2H), 7.78 (d, 1H), 8.14 (s, 1H), 8.83 (s, 1H), 10.14 (t, 1H).

### Beispiel 29

### 4-[3-{1-Cyclopropyl-[(2,4-dichlorbenzyl)amino]carbonyl}-1-fluor-8-methoxy-4-oxo-1,4-dihydrochinolin-7-yl]-(2RS,6SR)-2,6-dimethylpiperazin-1-yl}ethansäure - Hydrochlorid

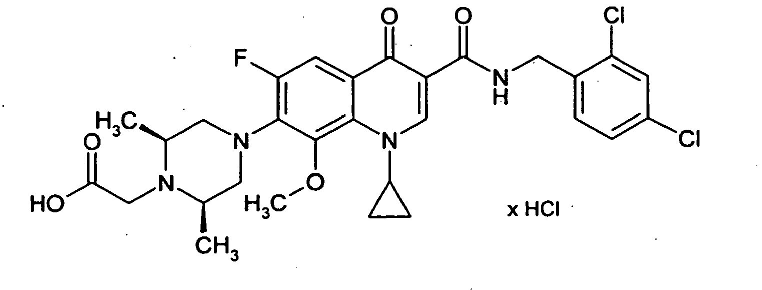

200 mg der Verbindung aus Beispiel 9 werden in 5 ml Dioxan gelöst, anschließend mit 5 ml 1M Lithiumhydroxid-Lösung versetzt und 2 h bei 50°C nachgerührt. Zur Aufarbeitung wird das Lösungsmittel am Rotationsverdampfer entfernt, der Rückstand mit Wasser aufgenommen, mit 1M Salzsäure angesäuert (pH 3-4). Der ausfallende Niederschlag wird abfiltriert, mit Wasser nachgewaschen und im Hochvakuum getrocknet. Man erhält 140 mg (73% d. Th.) der Titelverbindung.

LC-MS (Methode 3): Rₜ = 2.10 min, MS (ESI): m/z = 605 (M+H)⁺.

¹H-NMR (300 MHz, DMSO-d₆): δ = 0.99 (m, 2H), 1.18 (m, 2H), 1.38 (d, 6H), 3.46 (m, 2H), 3.55 (m, 2H), 3.70 (s, 3H), 3.78 (m, 4H), 3.95 (m, 1H), 4.68 (d, 2H), 7.20 (dd, 1H), 7.38 (m, 2H), 7.86 (d, 1H), 1H), 8.84 (s, 1H), 10.28 (t, 1H).

Analog zu Beispiel 28 werden nach der allgemeinen Arbeitsvorschrift 2 die in der nachfolgenden Tabelle aufgeführten Beispiele 30 bis 49 erhalten.

| **Beispiel-Nr.** | **Struktur** | **Edukte** | **Analytikdaten** |
|---|---|---|---|
| | | **Bsp.-Nr.** | **LC-MS (Methode)/ Meßwerte HPLC (Methode)/ Meßwert MS (Methode)/ Meßwert** |
| **30** | 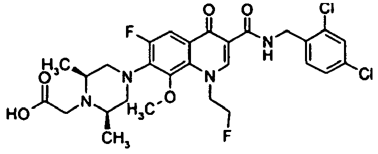 | 5 | HPLC (Methode 9): Rₜ = 4.41 min MS (ESI): m/z = 611 (M+H)⁺ |
| **31** | 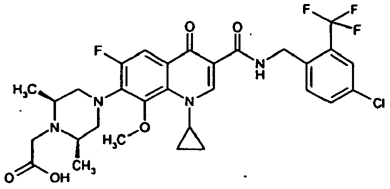 | 12 | LC-MS (Methode 3): Rₜ = 2.20 min MS (ESI): m/z = 639 (M+H)⁺ |
| **32** | 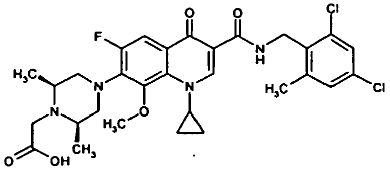 | 8 | LC-MS (Methode 3): Rₜ = 2.20 min MS (ESI): m/z = 619 (M+H)⁺ |
| **33** | 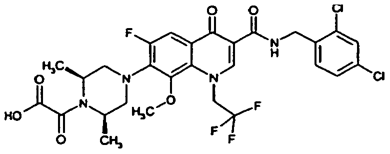 | 23 | LC-MS (Methode 3): Rₜ = 3.19 min MS (ESI): m/z = 689 (M+H)⁺ |
| **34** | 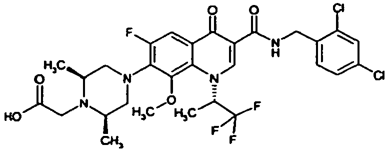 | 6 | HPLC (Methode 9): Rₜ = 4.69 min MS (ESI+): m/z = 662 (M+H)⁺ |
| **35** | 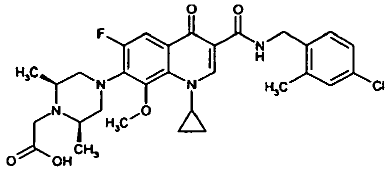 | 14 | LC-MS (Methode 3): Rₜ = 2.07 min MS (ES+): m/z = 585 (M+H)⁺ |
| **36** | 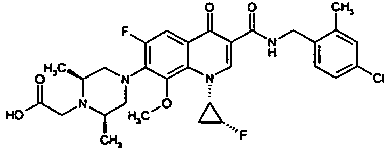 | 13 | LC-MS (Methode 3): **Rₜ** = 2.07 min MS (ESI+): m/z = 602 (M+H)⁺ |
| **37** | 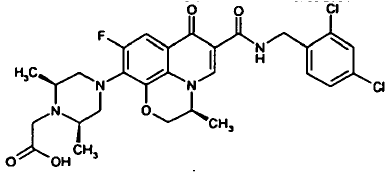 | 3 | LC-MS (Methode 3): Rₜ = 2.19 min MS (ESI): m/z = 619 (M+H)⁺ |
| **38** | 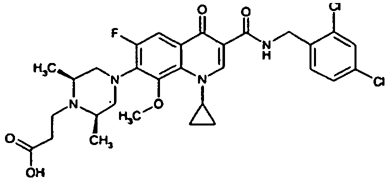 | 24 | LC-MS (Methode 1): **Rₜ** = 1.93 min MS (ES+): m/z = 619 (M+H)⁺ |
| **39** | 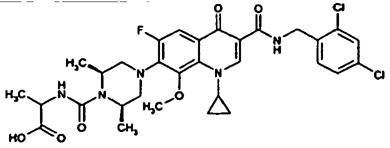 | 26 | LC-MS (Methode 1): Rₜ = 2.64 min MS (ES+): m/z = 662 (M+H)⁺ |
| **40** | 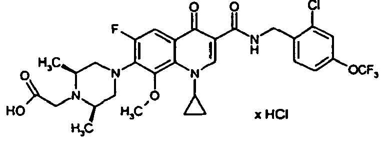 | 15 | LC-MS (Methode 1): Rₜ = 2.21 min MS (ES+): m/z = 655 (M+H)⁺ |
| **41** | 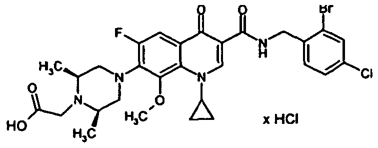 | 16 | LC-MS (Methode 1): Rₜ = 2.13 min MS (ES+): m/z = 649/651 (M+H)⁺ |
| **42** | 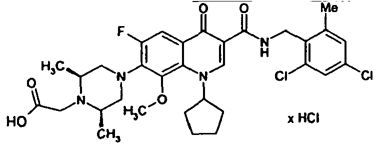 | 17 | LC-MS (Methode 1): Rₜ = 2.36 min MS (ES+): m/z = 647 (M+H)⁺ |
| **43** | 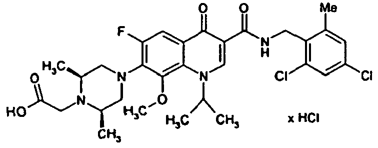 | 18 | LC-MS (Methode 1): **Rₜ** = 2.22 min MS (ES+): m/z = 621 (M+H)⁺ |
| **44** | 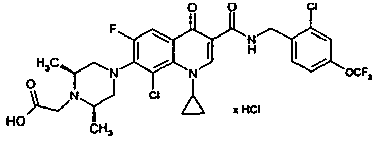 | 19 | LC-MS (Methode 1): Rₜ = 2.23 min MS (ES+): m/z = 659 (M+H)⁺ |
| **45** | 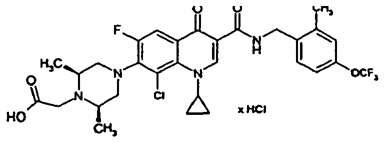 | 20 | LC-MS (Methode 1): Rₜ = 2.18 min MS (ES+): m/z = 639 (M+H)⁺ |
| **46** | 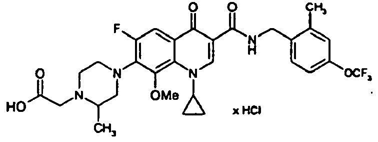 | 21 | LC-MS (Methode 1): **Rₜ** = 2.13 min MS (ES+): m/z = 621 (M+H)⁺ |
| **47** | 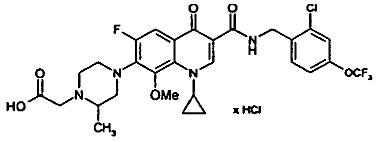 | 22 | LC-MS (Methode 1): Rₜ = 2.17 min MS (ES+): m/z = 641 (M+H)⁺ |
| **48** | 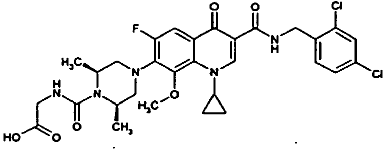 | 25 | LC-MS (Methode 3): Rₜ = 2.88 min MS (ES+): m/z = 676 (M+H)⁺ |
| **49** | 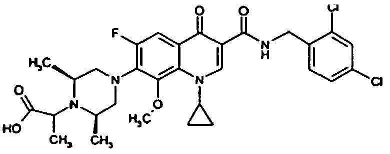 | 27 | HPLC (Methode 9): Rₜ = 4.31 min MS (ESI): m/z = 619 (M+H)⁺ |

### Beispiel 50

### 2-[(2RS,6SR)-4-(1-Cyclopropyl-3-{[(2,4-dichlorbenzyl)amino}carbonyl}-6-fluor-8-methoxy-4-oxo-1,4-dihydrochinolin-7-yl)-2,6-dimethylpiperazin-1-yl]-2-methylpropansäureethylester

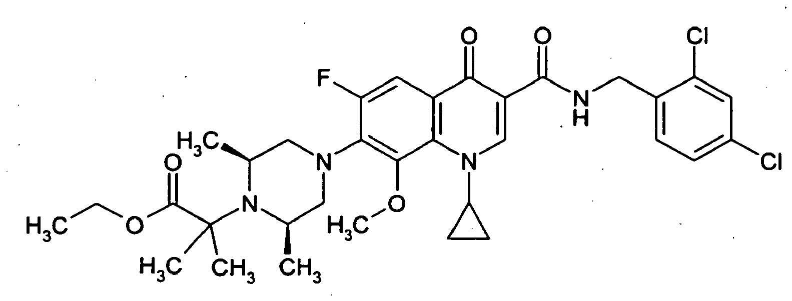

Die Titelverbindung wird analog zu Beispiel 27 aus der Verbindung aus Beispiel 66A und 2,4-Dichlorbenzylamin hergestellt.

LC-MS (Methode 1): Rₜ = 3.02 min.

MS (ES+): m/z = 661 (M+H)⁺.

### B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cytomegalo-Virus) Zytopathogenitätstests

Die Testverbindungen werden als 50 millimolare (mM) Lösungen in Dimethysulfoxid (DMSO) eingesetzt. Ganciclovir^{®}, Foscarnet^{®} und Cidofovir^{®} dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5% CO₂ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem O-verhead-Mikroskop (Plaque Multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50% im Vergleich zur unbehandelten Viruskontrolle hemmt;
SI (Selektivitätsindex) = CC₅₀ (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel-Nr.** | **NHDF CC₅₀ [µM]** | **HCMV EC₅₀ [µM]** | **SI** **HCMV** |
|---|---|---|---|
| **1** | 11.0 | 0.019 | 579 |
| **28** | 18.0 | 0.0023 | 8032 |
| **29** | 47.0 | 0.01 | 4700 |
| **30** | 47.0 | 0.008 | 5875 |
| **36** | 24.0 | 0.005 | 6402 |
| **49** | 94.0 | 0.018 | 5222 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

### Tiere:

5-6 Wochen alte immundefiziente Mäuse (16 - 20 g), Fox Chase SCID.NOD oder NOD.CB17-Prkdc/J werden von kommerziellen Züchtern (Taconic M&B, Dänemark; Jackson, USA) bezogen. Die Tiere werden unter sterilen Bedingungen (einschließlich Streu und Futter) in Isolatoren gehalten.

### Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01-0.03 werden die virusinfizierten Zellen 5-10 Tage später geerntet und in Gegenwart von Minimal Essential Medium (MEM), 20% foetalem Kälberserum (FKS) (v/v), 1% Glutamin (v/v), 1% Pen/Strep (v/v) mit 10% DMSO bei -80°C aufbewahrt. Nach serieller Verdünnung der virusinfizierten Zellen in Zehnerschritten erfolgt die Titerbestimmung auf 24-Well-Platten konfluenter NHDF-Zellen nach Fixierung und Färbung mit einer Giemsa-Formaldehyd-Lösung.

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM, 10% FKS (v/v), 1% Glutamin (v/v), 1% Pen/Strep (v/v) aufbewahrt. 1 x 10⁶ virusinfizierte NHDF-Zellen (Infektion mit HCMV-Davis oder HCMV AD169 M.O.I = 0.03) werden 3 Stunden nach Infektion abgelöst und in 20 µl MEM, 10% FKS (v/v), 1% Glutamin (v/v), 1% Pen/Strep (v/v) auf einen feuchten Schwamm getropft. Die Schwämme werden 3-4 Stunden inkubiert, um das adherieren der Zellen zu ermöglichen. Anschließend werden die Schwämme nach Zugabe von Medium (MEM, 10% FKS) (v/v), 1% Glutamin (v/v), 1% Pen/Strep (v/v)) über Nacht inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber oder Klammern verschlossen. 4 - 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (9 Uhr) peroral mit Substanz behandelt. Die Tagesdosis beträgt beispielsweise 1 oder 3 oder 10 oder 30 oder 100 mg/kg Körpergewicht, das Applikationsvolumen ist 10 ml/kg Körpergewicht. Die Formulierung der Substanzen erfolgt in Form einer 0.5%-igen Tylosesuspension / PBS mit 2% DMSO oder einer anderen geeigneten Mischung, die die Löslichkeit der Substanzen unterstützt, z. B. 2 % Ethanol, 2.5% Solutol, 95.5% PBS. 10 Tage nach Transplantation und ca. 16 Stunden nach der letzten Substanzapplikation werden die Tiere schmerzlos getötet und der Schwamm entnommen. Die virusinfizierten Zellen werden durch Kollagenaseverdau (330 U/ 1.5 ml) aus dem Schwamm freigesetzt und in Gegenwart von MEM, 10% FKS (v/v), 1% Glutamin (v/v), 1% Pen/Strep (v/v), 10% DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Fixierung und Färbung mit einer Giemsa-Formaldehyd-Lösung. Ermittelt wird die Anzahl infizierter Zellen bzw. infektiöser Viruspartikel (infectious center assay) nach Substanzbehandlung im Vergleich zur placebobehandelten Kontrollgruppe. Die statistische Auswertung erfolgt mittels geeigneter Computerprogramme, z. B. GraphPad Prism.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung :

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

10-500 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 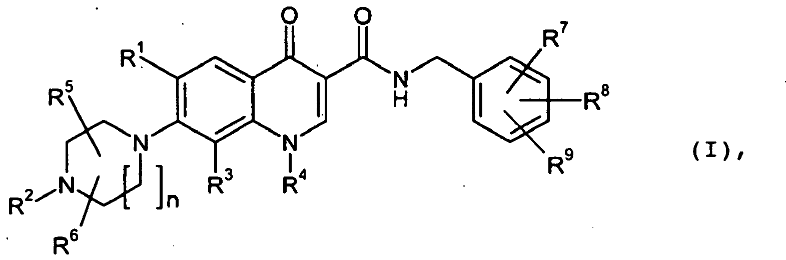 in welcher
n für eine Zahl 1 oder 2 steht,
R¹ für Wasserstoff, Fluor, Chlor oder Trifluormethyl steht,
R² für C₁-C₆-Alkyl, C₁-C₆-Alkylaminocarbonyl oder -C(=O)-R¹⁰ steht,
wobei Alkyl und Alkylaminocarbonyl substituiert sind mit einem Substituenten, wobei der Substituent ausgewählt wird aus der Gruppe, bestehend aus Hydroxycarbonyl und C₁-C₆-Alkoxycarbonyl,
und
R¹⁰ für Hydroxycarbonyl, C₁-C₆-Alkoxycarbonyl, Hydroxycarbonylmethyl oder C₁-C₆-Alkoxycarbonylmethyl steht,
R³ für Halogen, Cyano, Methoxy, Trifluormethyl, Monofluormethoxy, Difluormethoxy, Trifluormethoxy oder Ethinyl steht,
R⁴ für C₁-C₆-Alkyl oder C₃-C₈-Cycloalkyl steht,
wobei Alkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkoxy, C₁-C₆-Alkylamino, C₁-C₆-Alkylcarbonyl und C₁-C₆-Alkoxycarbonyl,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Amino, Cyano, Trifluormethyl, Hydroxycarbonyl, Aminocarbonyl, C₁-C₆-Alkyl, C₁-C₆-Alkoxy, C₁-C₆-Alkylamino, C₁-C₆-Alkylcarbonyl und C₁-C₆-Alkoxycarbonyl,
oder
R³ und R⁴ bilden gemeinsam mit den Atomen an die sie gebunden sind einen Ring durch eine Gruppe der Formel 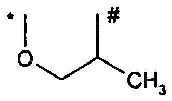 wobei
* die Anknüpfstelle an das Kohlenstoffatom ist, und
# die Anknüpfstelle an das Stickstoffatom ist,
R⁵ und R⁶ unabhängig voneinander für Wasserstoff, Methyl oder Ethyl stehen,
R⁷ und R⁸ unabhängig voneinander für Halogen, Hydroxy, Cyano, Trifluormethyl, Monofluormethoxy, Difluormethoxy, Trifluormethoxy, C₁-C₃-Alkyl oder C₁-C₃₋Alkoxy stehen,
R⁹ für Wasserstoff, Halogen, Hydroxy, Cyano, Trifluormethyl, Monofluormethoxy, Difluormethoxy, Trifluormethoxy, C₁-C₃-Alkyl oder C₁-C₃-Alkoxy steht,
oder eines ihrer Salze, ihrer Solvate oder der Solvate ihrer Salze.

2. Verbindung nach Anspruch 1, **dadurch gekennzeichnet, dass** sie der Formel 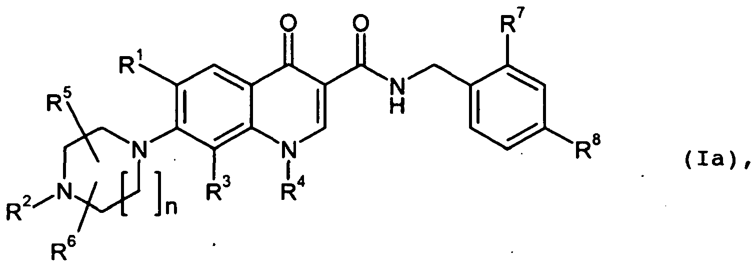 entspricht,
in welcher
n für die Zahl 1 steht,
R¹ für Wasserstoff oder Fluor steht,
R² für C₁-C₄-Alkyl steht,
wobei Alkyl substituiert ist mit einem Substituenten, wobei der Substituent ausgewählt wird aus der Gruppe, bestehend aus Hydroxycarbonyl und C₁-C₆-Alkoxycarbonyl,
R³ für Fluor, Chlor, Trifluormethyl, Methoxy, Monofluormethoxy, Difluormethoxy, Trifluormethoxy oder Ethinyl steht,
R⁴ für C₁-C₄-Alkyl oder C₃-C₅-Cycloalkyl steht,
wobei Alkyl substituiert sein kann mit 1 bis 3 Substituenten, wobei die Substituenten unabhängig von- einander ausgewählt werden aus der Gruppe, bestehend aus Fluor, Hydroxy und C₁-C₃-Alkoxy,
und
wobei Cycloalkyl substituiert sein kann mit 1 bis 3 Substituenten, wobei die Substituenten unabhängig voneinander ausgewählt werden aus der Gruppe, bestehend aus Halogen, Hydroxy, Trifluormethyl, C₁-C₃-Alkyl und C₁-C₃-Alkoxy, oder
R³ und R⁴ bilden gemeinsam mit den Atomen an die sie gebunden sind einen Ring durch eine Gruppe der Formel 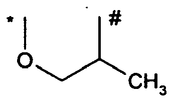 wobei
* die Anknüpfstelle an das Kohlenstoffatom ist, und
# die Anknüpfstelle an das Stickstoffatom ist,
R⁵ und R⁶ unabhängig voneinander für Wasserstoff oder Methyl stehen,
R⁷ und R⁸ unabhängig voneinander für Fluor, Chlor, Cyano, Trifluormethyl, Difluormethoxy, Trifluormethoxy, C₁-C₃-Alkyl oder C₁-C₃-Alkoxy stehen,
oder eines ihrer Salze, ihrer Solvate oder der Solvate ihrer Salze.

3. Verbindung nach Anspruch 1 oder 2, **dadurch gekennzeichnet, dass** sie der Formel 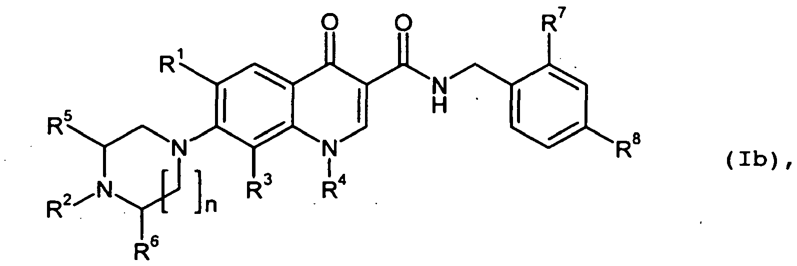 entspricht
in welcher
n für die Zahl 1 steht,
R¹ für Fluor steht,
R² für Methyl oder Ethyl steht,
wobei Methyl und Ethyl substituiert sind mit einem Substituenten, wobei der Substituent ausgewählt wird aus der Gruppe, bestehend aus Hydroxycarbonyl, Methoxycarbonyl und Ethoxycarbonyl,
R³ für Chlor, Methoxy, Difluormethoxy oder Trifluormethoxy steht,
R⁴ für Methyl, Ethyl oder Cyclopropyl steht, wobei Ethyl substituiert sein kann mit 1 bis 3 Substituenten Fluor,
und
wobei Cyclopropyl substituiert sein kann mit 1 bis 2 Substituenten Fluor,
oder
R³ und R⁴ bilden gemeinsam mit den Atomen an die sie gebunden sind einen Ring durch eine Gruppe der Formel 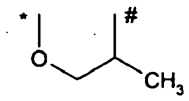 wobei
* die Anknüpfstelle an das Kohlenstoffatom ist, und
# die Anknüpfstelle an das Stickstoffatom ist,
R⁵ und R⁶ unabhängig voneinander für Wasserstoff oder Methyl stehen,
R⁷ und R⁸ unabhängig voneinander für Chlor, Trifluormethyl, Trifluormethoxy oder Methyl stehen,
oder eines ihrer Salze, ihrer Solvate oder der Solvate ihrer Salze.

4. Verfahren zur Herstellung einer Verbindung der Formel (I) nach Anspruch 1, **dadurch gekennzeichnet, dass** eine Verbindung der Formel 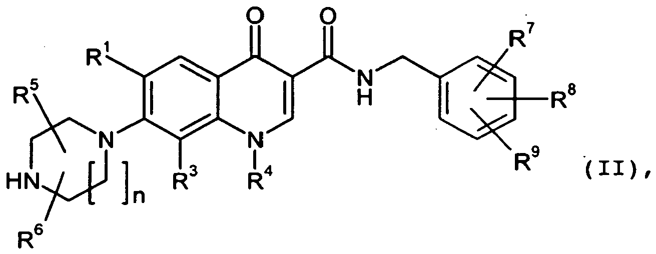 in welcher
n, R¹, R³, R⁴, R⁵, R⁶, R⁷ , R⁸ und R⁹ die in Anspruch 1 angegebene Bedeutung aufweisen,
nach Verfahren [A] mit einer Verbindung der Formel
R²-X¹ (III),
in welcher
R² für C₁-C₆-Alkyl steht, wobei Alkyl substituiert ist mit einem Substituenten C₁-C₆-Alkoxycarbonyl,
und
X¹ für Halogen, bevorzugt Iod, Chlor oder Brom, oder Mesylat, Tosylat oder Triflat steht,
oder
nach Verfahren [B] mit einer Verbindung der Formel
R^{2a}-NCO (IV),
in welcher
R^{2a} für das Alkyl von Alkylaminocarbonyl des Restes R² steht,
wobei Alkylaminocarbonyl substituiert ist mit einem Substituenten C₁-C₆-Alkoxycarbonyl,
oder
nach Verfahren [C] mit einer Verbindung der Formel 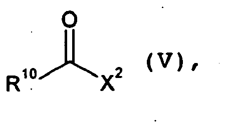 in welcher
R¹⁰ die in Anspruch 1 angegebene Bedeutung aufweist,
und
X² für Halogen, bevorzugt Chlor oder Brom steht,
umgesetzt wird,
oder
wobei eine Verbindung, die durch die Umsetzung von einer Verbindung der Formel (II) mit einer Verbindung der Formeln (III) oder (IV) entsteht,
nach Verfahren [D] mit einer Base zu der entsprechenden Säure verseift wird.

5. Verbindung nach einem der Ansprüche 1 bis 3 zur Behandlung und/oder Prophylaxe von Krankheiten.

6. Arzneimittel enthaltend eine Verbindung nach einem der Ansprüche 1 bis 3 in Kombination mit einem inerten, nichttoxischen, pharmazeutisch geeigneten Hilfsstoff.

7. Verwendung einer Verbindung nach einem der Ansprüche 1 bis 3 zur Herstellung eines Arzneimittels zur Behandlung und/oder Prophylaxe von Virusinfektionen.

8. Verwendung nach Anspruch 7, **dadurch gekennzeichnet, dass** die Virusinfektion eine Infektion mit dem humanen Cytomegalovirus (HCMV) oder einem anderen Vertreter der Gruppe der Herpes viridae ist.

9. Arzneimittel nach Anspruch 6 zur Behandlung und/oder Prophylaxe von Virusinfektionen.

10. Verwendung einer antiviral wirksamen Menge einer Verbindung nach einem der Ansprüche 1 bis 3, eines Arzneimittels nach Anspruch 6 oder eines nach Anspruch 7 oder 8 erhaltenen Arzneimittels zur Herstellung eines Arzneimittels zur Bekämpfung von Virusinfektionen in Menschen und Tieren.

## Claims

1. Compound of formula 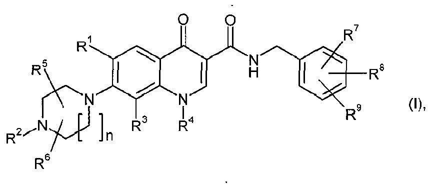 in which
n represents a number 1 or 2,
R¹ represents hydrogen, fluorine, chlorine or trifluoromethyl,
R² represents C₁-C₆-alkyl, C₁-C₆-alkylaminocarbonyl or -C(=O)-R¹⁰,
whereby alkyl and alkylaminocarbonyl are substituted with a substituent, whereby the substituent is selected from the group consisting of hydroxycarbonyl and C₁-C₆-alkoxycarbonyl,
and
R¹⁰ represents hydroxycarbonyl, C₁-C₆-alkoxycarbonyl, hydroxycarbonylmethyl or C₁-C₆-alkoxycarbonylmethyl,
R³ represents halogen, cyano, methoxy, trifluoromethyl, monofluoromethoxy, difluoromethoxy, trifluoromethoxy or ethynyl,
R⁴ represents C₁-C₆-alkyl or C₃-C₈-cycloalkyl,
whereby alkyl can be substituted with 1 to 3 substituents, whereby the substituents are selected independently of one another from the group consisting of halogen, hydroxy, amino, cyano, trifluoromethyl, hydroxycarbonyl, aminocarbonyl, C₁-C₆-alkoxy, C₁-C₆-alkylamino, C₁-C₆-alkylcarbonyl and C₁-C₆-alkoxycarbonyl,
and
whereby cycloalkyl can be substituted with 1 to 3 substituents, whereby the substituents are selected independently of one another from the group consisting of halogen, hydroxy, amino, cyano, trifluoromethyl, hydroxycarbonyl, aminocarbonyl, C₁-C₆-alkyl, C₁-C₆-alkoxy, C₁-C₆-alkylamino, C₁-C₆-alkylcarbonyl and C₁-C₆-alkoxycarbonyl,
or
R³ and R⁴, together with the atoms to which they are bonded, form a ring through a group of formula 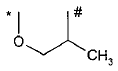 whereby
* is the linkage site to the carbon atom, and
# is the linkage site to the nitrogen atom,
R⁵ and R⁵ independently of one another represent hydrogen, methyl or ethyl,
R⁷ and R⁸ independently of one another represent halogen, hydroxy, cyano, trifluoromethyl, monofluoromethoxy, difluoromethoxy, trifluoromethoxy, C₁-C₃-alkyl or C₁-C₃-alkoxy,
R⁹ represents hydrogen, halogen, hydroxy, cyano, trifluoromethyl, monofluoromethoxy, difluoromethoxy, trifluoromethoxy, C₁-C₃-alkyl or C₁-C₃-alkoxy,
or one of its salts, its solvates or the solvates of its salts.

2. Compound according to Claim 1, **characterized in that** it corresponds to formula 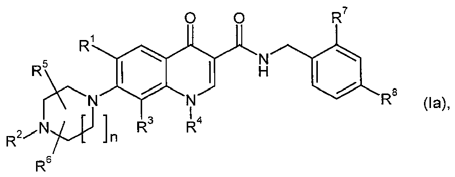 in which
n represents the number 1,
R' represents hydrogen or fluorine,
R² represents C₁-C₄-alkyl,
whereby alkyl is substituted with a substituent, whereby the substituent is selected from the group consisting of hydroxycarbonyl and C₁-C₆-alkoxycarbonyl,
R³ represents fluorine, chlorine, trifluoromethyl, methoxy, monofluoromethoxy, difluoromethoxy, trifluoromethoxy or ethynyl,
R⁴ represents C₁-C₄-alkyl or C₃-C₅-cycloalkyl,
whereby alkyl can be substituted with 1 to 3 substituents, whereby the substituents are selected independently of one another from the group consisting of fluorine, hydroxy and C₁-C₃-alkoxy,
and
whereby cycloalkyl can be substituted with 1 to 3 substituents, whereby the substituents are selected independently of one another from the group consisting of halogen, hydroxy, trifluoromethyl, C₁-C₃-alkyl and C₁-C₃-alkoxy,
or
R³ and R⁴, together with the atoms to which they are bonded, form a ring through a group of formula 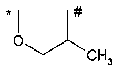 whereby
* is the linkage site to the carbon atom,
and
# is the linkage site to the nitrogen atom,
R⁵ and R⁶ independently of one another represent hydrogen or methyl,
R⁷ and R⁸ independently of one another represent fluorine, chlorine, cyano, trifluoromethyl, difluoromethoxy, trifluoromethoxy, C₁-C₃-alkyl or C₁-C₃-alkoxy,
or one of its salts, its solvates or the solvates of its salts.

3. Compound according to Claim 1 or 2, **characterized in that** it corresponds to formula 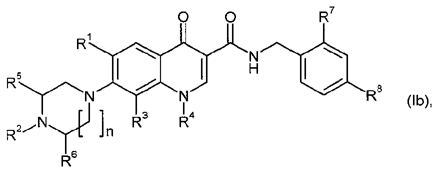 in which
n represents the number 1,
R¹ represents fluorine,
R² represents methyl or ethyl,
whereby methyl and ethyl are substituted with a substituent, whereby the substituent is selected from the group consisting of hydroxycarbonyl, methoxycarbonyl and ethoxycarbonyl,
R³ represents chlorine, methoxy, difluoromethoxy or trifluoromethoxy,
R⁴ represents methyl, ethyl or cyclopropyl,
whereby ethyl can be substituted with 1 to 3 fluorine substituents,
and
whereby cyclopropyl can be substituted with 1 to 2 fluorine substituents,
or
R³ and R⁴, together with the atoms to which they are bonded, form a ring through a group of formula 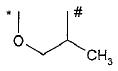 whereby
* is the linkage site to the carbon atom, and
# is the linkage site to the nitrogen atom,
R⁵ and R⁶ independently of one another represent hydrogen or methyl,
R⁷ and R⁸ independently of one another represent chlorine, trifluoromethyl, trifluoromethoxy or methyl,
or one of its salts, its solvates or the solvates of its salts.

4. Method for preparing a compound of formula (I) according to Claim 1, **characterized in that** a compound of formula 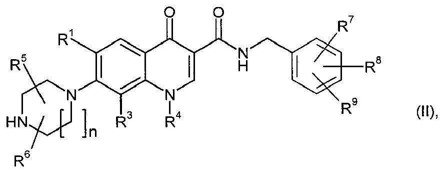 in which
n, R¹, R³, R⁴, R⁵, R⁶, R⁷, R⁸ and R⁹ have the meaning indicated in Claim 1,
is reacted,
according to method [A], with a compound of formula
R²-X¹ (III),
in which
R² represents C₁-C₆-alkyl,
whereby alkyl is substituted with a substituent C₁-C₆-alkoxycarbonyl
and X¹ represents halogen, preferably iodine, chlorine or bromine, or mesylate, tosylate or triflate,
or
according to method [B], with a compound of formula
R^{2a}-NCO (IV),
in which
R^{2a} represents the alkyl of the alkylaminocarbonyl of the radical R²,
whereby alkylaminocarbonyl is substituted with a substituent C₁-C₆-alkoxycarbonyl,
or
according to method [C], with a compound of formula 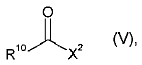 in which
R¹⁰ has the meaning indicated in Claim 1,and
X² represents halogen, preferably chlorine or bromine,
or
whereby a compound formed by the reaction of a compound of formula (II) with a compound of formulae (III) or (IV)
is hydrolysed, according to method [D], with a base to form the corresponding acid.

5. Compound according to any one of Claims 1 to 3 for the treatment and/or prophylaxis of diseases.

6. Medicament comprising a compound according to any one of Claims 1 to 3 in combination with an inert, non-toxic, pharmaceutically acceptable excipient.

7. Use of a compound according to any one of Claims 1 to 3 for the production of a medicament for the treatment and/or prophylaxis of viral infections.

8. Use according to Claim 7, **characterized in that** the viral infection is an infection with the human cytomegalovirus (HCMV) or another representative of the group of herpes viridae.

9. Medicament according to Claim 6 for the treatment and/or prophylaxis of viral infections.

10. Use of an antivirally effective amount of a compound according to any one of Claims 1 to 3, of a medicament according to Claim 6 or of a medicament obtained according to Claim 7 or 8 for the production of a medicament for controlling viral infections in humans and animals.

## Revendications

1. Composé de formule 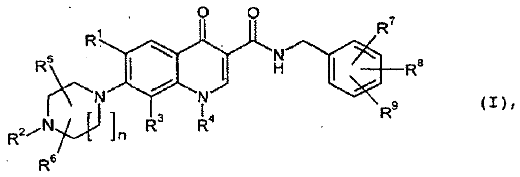 dans laquelle
n représente le nombre 1 ou 2,
R¹ représente l'hydrogène, le fluor, le chlore ou le trifluorométhyle,
R² est un radical alkyle en C₁-C₆, (alkyle en C₁-C₆)aminocarbonyle ou -C(=O)-R¹⁰,
où les radicaux alkyle et alkylaminocarbonyle sont substitués par un substituant, le substituant étant choisi dans le groupe consistant en les substituants hydroxycarbonyle et (alcoxy en C₁-C₆)carbonyle ; et R¹⁰ est un radical hydroxycarbonyle, (alcoxy en C₁-C₆)carbonyle, hydroxycarbonylméthyle ou (alcoxy en C₁-C₆)carbonylméthyle,
R³ est un groupe halogéno, cyano, méthoxy, trifluorométhyle, monofluorométhoxy, difluorométhoxy, trifluorométhoxy ou éthynyle,
R⁴ représente un radical alkyle en C₁-C₆ ou cycloalkyle en C₃-C₈,
le radical alkyle pouvant être substitué par 1 à 3 substituants, les substituants étant choisis indépendamment les uns des autres dans le groupe consistant en les substituants halogéno, hydroxy, amino, cyano, trifluorométhyle, hydroxycarbonyle, aminocarbonyle, alcoxy en C₁-C₆, (alkyle en C₁-C₆)amino, (alkyle en C₁-C₆)carbonyle et (alcoxy en C₁-C₆)carbonyle ;
et le radical cycloalkyle pouvant être substitué par 1 à 3 substituants, les substituants étant choisis indépendamment les uns des autres dans le groupe consistant en les substituants halogéno, hydroxy, amino, cyano, trifluorométhyle, hydroxycarbonyle, aminocarbonyle, alkyle en C₁-C₆, alcoxy en C₁-C₆, (alkyle en C₁-C₆)amino, (alkyle en C₁-C₆)carbonyle et (alcoxy en C₁-C₆)carbonyle,
ou
R³ et R⁴, avec les atomes auxquels ils sont liés, forment un cycle grâce à un groupe de formule 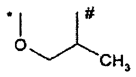 dans laquelle * est le point de liaison à l'atome de carbone, et # est le point de liaison à l'atome d'azote,
R⁵ et R⁶ représentent chacun indépendamment de l'autre un atome d'hydrogène, le groupe méthyle ou éthyle,
R⁷ et R⁸ représentent chacun indépendamment de l'autre un halogène, un groupe hydroxy, cyano, trifluorométhyle, monofluorométhoxy, difluorométhoxy ou trifluorométhoxy, un radical alkyle en C₁-C₃ ou alcoxy en C₁-C₃,
R⁹ représente un atome d'hydrogène, un halogène, un groupe hydroxy, cyano, trifluorométhyle, monofluorométhoxy, difluorométhoxy ou trifluorométhoxy, un radical alkyle en C₁-C₃ ou alcoxy en C₁-C₃,
ou l'un de ses sels, de ses produits de solvatation ou des produits de solvatation de ses sels.

2. Composé selon la revendication 1, **caractérisé en ce qu'**il a la formule 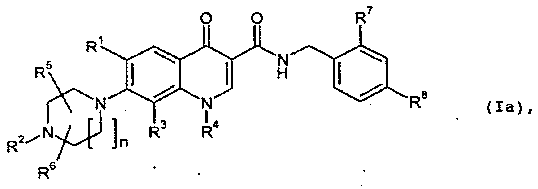 dans laquelle
n représente le nombre 1,
R¹ représente l'hydrogène ou le fluor,
R² est un radical alkyle en C₁-C₄, le radical alkyle étant substitué par un substituant, le substituant étant choisi dans le groupe consistant en les substituants hydroxycarbonyle et (alcoxy en C₁-C₆)carbonyle,
R³ représente le fluor, le chlore trifluorométhyle, méthoxy, monofluorométhoxy, difluorométhoxy, trifluorométhoxy ou éthynyle,
R⁴ est un radical alkyle en C₁-C₄ ou cycloalkyle en C₃-C₅, le radical alkyle pouvant être substitué par 1 à 3 substituants, les substituants étant choisis indépendamment les uns des autres dans le groupe consistant en les substituants fluoro, hydroxy et alcoxy en C₁-C₃ ; et le radical cycloalkyle pouvant être substitué par 1 à 3 substituants, les substituants étant choisis indépendamment les uns des autres dans le groupe consistant en les substituants halogéno, hydroxy, trifluorométhyle, alkyle en C₁-C₃ et alcoxy en C₁-C₃,
ou
R³ et R⁴, avec les atomes auxquels ils sont liés, forment un cycle grâce à un groupe de formule 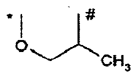 dans laquelle * est le point de liaison à l'atome de carbone et # est le point de liaison à l'atome d'azote,
R⁵ et R⁶ représentent chacun indépendamment de l'autre un atome d'hydrogène ou le groupe méthyle,
R⁷ et R⁸ représentent chacun indépendamment de l'autre le fluor, le chlore, cyano, trifluorométhyle, difluorométhoxy, trifluorométhoxy, un radical alkyle en C₁-C₃ ou alcoxy en C₁-C₃,
ou l'un de ses sels, de ses produits de solvatation ou des produits de solvatation de ses sels.

3. Composé selon la revendication 1 ou 2, **caractérisé en ce qu'**il a la formule 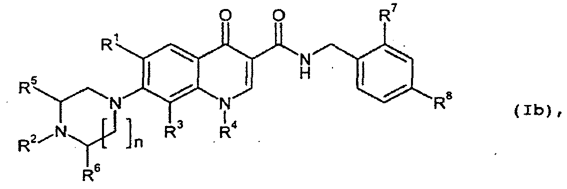 dans laquelle
n représente le nombre 1,
R¹ est le groupe fluoro,
R² est le groupe méthyle ou éthyle,
les groupes méthyle et éthyle étant substitués par un substituant, le substituant étant choisi dans le groupe consistant en les substituants hydroxycarbonyle, méthoxycarbonyle et éthoxycarbonyle,
R³ représente le chlore, méthoxy, difluorométhoxy ou trifluorométhoxy,
R⁴ est le groupe méthyle, éthyle ou cyclopropyle, le groupe éthyle pouvant être substitué par 1 à 3 substituants fluor, et le groupe cyclopropyle pouvant être substitué par 1 à 2 substituants fluor,
ou
R³ et R⁴, avec les atomes auxquels ils sont liés, forment un cycle grâce à un groupe de formule 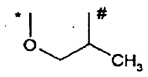 dans laquelle * est le point de liaison à l'atome de carbone, et # est le point de liaison à l'atome d'azote,
R⁵ et R⁶ représentent chacun indépendamment de l'autre un atome d'hydrogène ou le groupe méthyle,
R⁷ et R⁸ représentent chacun indépendamment de l'autre le chlore, trifluorométhyle, trifluorométhoxy ou méthyle,
ou l'un de ses sels, de ses produits de solvatation, ou des produits de solvatation de ses sels.

4. Procédé de préparation d'un composé de formule (I) selon la
revendication 1, **caractérisé en ce qu'**on fait réagir un composé de formule 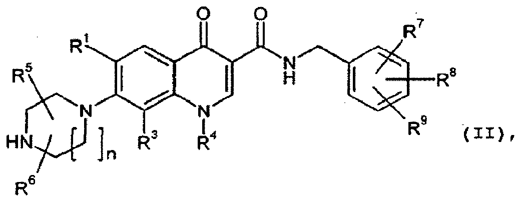 dans laquelle n, R¹, R³, R⁴, R⁵, R⁶, R⁷, R⁸ et R⁹ ont les significations données dans la revendication 1,
d'après le procédé [A], avec un composé de formule
R²-X¹ (III)
dans laquelle R² est un radical alkyle en C₁-C₆, le radical alkyle étant substitué par un substituant (alcoxy en C₁-C₆)carbonyle, et X¹ représente un halogène, de préférence l'iode, le chlore ou le brome, ou le mésylate, le tosylate ou le triflate,
ou
d'après le procédé [B], avec un composé de formule
R^{2a}-NCO (IV)
dans laquelle R^{2a} représente le groupe alkyle du groupe alkylaminocarbonyle du radical R², le groupe alkylaminocarbonyle étant substitué par un substituant (alcoxy en C₁-C₆)carbonyle,
ou
d'après le procédé [C], avec un composé de formule 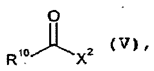 dans laquelle R¹⁰ a les significations données dans la revendication 1, et X² représente un halogène, de préférence le chlore ou le brome,
ou
dans lequel, d'après le procédé [D], on saponifie avec une base, pour obtenir l'acide correspondant, un composé qui se forme par la réaction d'un composé de formule (II) avec un composé de formule (III) ou (IV).

5. Composé selon l'une des revendications 1 à 3, pour le traitement et/ou la prophylaxie de maladies.

6. Médicament contenant un composé selon l'une des revendications 1 à 3 en combinaison avec un adjuvant inerte, non toxique, pharmaceutiquement acceptable.

7. Utilisation d'un composé selon l'une des revendications 1 à 3 pour fabriquer un médicament destiné au traitement et/ou à la prophylaxie d'infections virales.

8. Procédé selon la revendication 7, **caractérisé en ce que** l'infection virale est une infection par le cytomégalovirus humain (HCMV) ou un autre représentant du groupe des Herpes viridae.

9. Médicament selon la revendication 6, destiné au traitement et/ou à la prophylaxie d'infections virales.

10. Utilisation d'une quantité à effet antiviral d'un composé selon l'une des revendications 1 à 3, d'un médicament selon la revendication 6 ou d'un médicament obtenu selon la revendication 7 ou 8, pour fabriquer un médicament destiné à lutter contre les infections virales chez l'homme et l'animal.
